# EUROPEAN PATENT APPLICATION

(11) **EP 1 367 393 A1**
(43) Date of publication of application: **03.12.2003**
(21) Application number: 02077071.5
(22) Date of filing: 27.05.2002
(51) Int. Cl.: G01N 33/50, C07K 14/705, A61K 38/00

(54) **Methods for using the CD 163 pathway for modulating an immune response**

(71) Applicant: MacroZyme B.V., 1105 BA Amsterdam (NL)
(72) Inventor: Boon, Louis, 1069 NJ Amsterdam (NL); van Neerven, Ruprecht Jules Joost, 1321 BW Almere (NL)
(74) Representative: Prins, Adrianus Willem

(57) **Abstract**

The invention relates to the field of immunology, gene therapy and medicine. More specifically, the invention relates to the identification of a molecule capable of interacting with a soluble and/or cell bound form of CD163 and as a result of said interaction an immune response is either instigated or suppressed in an organism. Furthermore it relates to the preparation of a pharmaceutical composition comprising said molecule and/or antagonist I and/or agonist I thereof, and/or an isolated CD163 and/or an antagonist II or agonist II thereof, for the therapeutic or prophylactic treatment of an individual with an immune response disorder.

## Description

The invention relates to the field of immunology, gene therapy and medicine. More specifically, the invention relates to the identification of a molecule capable of interacting with a soluble and/or cell bound form of CD163 and as a result of said interaction an immune response is either instigated or suppressed in an organism. Furthermore it relates to the preparation of a pharmaceutical composition comprising said molecule and/or antagonist I and/or agonist I thereof, and/or an isolated CD163 and/or an antagonist II or agonist II thereof, for the therapeutic or prophylactic treatment of an individual with an immune related disorder.

Antigen presenting cells (APC) of the myeloid lineage such as monocytes, macrophages, and dendritic cells, are key regulators in innate and acquired immune responses. They are capable of capturing and processing antigens, and presenting them to T lymphocytes. The complete activation of T cells is dependent on the interaction of costimulating molecules on APC (eg. CD80, CD86) with their counterparts on T cells (CD28, CTLA-4). Upon activation via costimulatory molecules as CD40, monocytes, macrophages and dendritic cells are further capable of secreting cytokines like IL-12 and IL-10 that determine the type of immune response generated against a given pathogen. The cytokines secreted by APC are regulated by the activity of a range of receptors, known as pattern recognition receptors (PRR). These receptors are a key feature of the innate immune response because they help discriminate between self and infectious non-self. The PRR include the Toll-like receptors, mannose receptor, and scavenging receptors (Linehan et al. 2000, Imler and Hoffmann 2001). These receptors recognize conserved pathogen-associated molecular patterns, which are shared by large groups of microorganisms, and may also recognize endogenous ligands induced during inflammatory responses. As a result, when activated through PRR the APC are capable of initiating type 1 (inflammatory) versus type 2 (antibody mediated) immune responses (Kopp and Medzhitov 1999, Imler and Hoffmann 2001, Re and Strominger 2001).

One of these pattern recognition proteins, human CD163 (also recognized as M130 or RM3/1) was identified as a membrane protein that is expressed on macrophages and on monocytes (Morganelli et al 1988, Zwadlo et al 1987, Law et al 1993, Pulford et al 1992, Sulahian et al 2000). cDNAs encoding murine (Schaer et al. 2001) as well as human CD163 (Law et al. 1993, Hogger et al 1998, Ritter et al 1999) have been identified and have shown that CD163 is a member of the cystine-rich scavenger receptor (SRCR) protein superfamily type B. Several isoforms have been described that differ in the length of the cytoplasmic region (45, 84 and 89 amino acids respectively), suggesting that these isoforms may have differential signaling and function (Högger et al 1998, Law et al 1993, Ritter et al 1999). The CD163 gene encodes a 130 kDa transmembrane protein, which has recently been shown to be involved in hemoglobin (Hb) metabolism (Kristiansen et al, 2001). Efficient removal of free Hb is essential for health because of the oxidative and toxic properties of the iron-containing haem in Hb. The elevated expression of CD163 on tissue macrophages is therefore in line with its physiological important function in Hb catabolism of macrophages. Free Hb released into the serum is complexed by the acute phase protein haptoglobin and the complexes of haptoglobin and Hb are scavenged by CD163 that only recognizes the complex and not the components alone. Upon binding to CD163 complexes are endocytozed by the macrophages, whereupon the haem present in Hb is converted into bilirubin and iron.

Freshly isolated human monocytes have a low level expression of CD163. Upon *in- vitro* culture of such monocytes in the presence of macrophage colony-stimulating factor (M-CSF) the monocytes differentiate into macrophages. This is accompanied by up-regulation of CD163 mRNA and protein (Buechler et al, 2000). When monocytes are cultured in the presence of GM-CSF and IL-4, factors that induce differentiation into dendritic cells, CD163 expression is downregulated (Buechler et al, 2000). Interestingly, dendritic cells are the central cells involved in initiating immune responses *in vivo,* and therefore the rationale of the low expression levels of CD163 on these cells may be related to the strong immunostimulatory capacity of these cells.

Although (gluco)corticosteroids are the most widely used immunosuppressive and anti-inflammatory agent in current clinical medicine, their pharmacological activities involved in modulation of the immune system are poorly understood. Unlike the role of CD163 in Hb catabolism, scavenger receptor cysteine-rich (SRCR) proteins are typically associated with immune function and are therefore expressed on cells from the immune system. Interestingly, the expression of CD163 on monocytes and macrophages is upregulated *in vitro* by anti-inflammatory mediators such as (gluco)corticoisteroids and IL-6 and IL-10. Further, *in vivo* administration of corticosteroids to human volunteers resulted in an increase both in antigen density and the number of cells that express CD163, 6 hours after administration (Zwadlo-Klarwasser et al 1990). On the other hand, inflammatory mediators like lipopolysaccharide (LPS), IFN-γ and TNF-α suppress expression of CD163 on these cells.

Stimulation of macrophages by crosslinking CD163 with the monoclonal antibody EDHU-1 induces the production of the cytokines IL-1β, IL-6 and GM-CSF (Ritter et al 2001, van den Heuvel et al 1999). CD163 signal transmission upon stimulation depends on a protein tyrosine kinase (PTK) activity resulting in calcium mobilization and inositol triphosphate generation. Since the cytoplasmic domains of CD163 and its isoforms contain no known PTK motif, the molecular mechanisms by which CD163 signal transduction is mediated are not understood. In addition to the PTK-dependent signal another non characterized signal pathway has been described that is independent of PTK activity. More recently, interaction with the regulatory subunit of casein kinase II and protein kinase C with the cytoplasmic tail of CD163 on macrophages have been observed (Ritter et al, 2001).

Further, immunohistochemical analysis has shown that CD163-positive macrophages accumulate in inflammatory sites during the healing phase of both acute and chronic inflammation, suggesting a role for CD163-positive macrophages with the healing phase of the inflammatory response (Zwadlo et al 1987). In contrast, in other papers, the occurrence of CD163 and macrophages preceded the onset of inflammation (Polfiet et al., 2002, Baeten et al., 2002). Also Moller et al., 2002 concluded that the increased levels of sCD163 (i.e. CD163 soluble form) in patients with myelomonocytic leukemias and infections, is due to increased proliferation of cells of the myelomonocytic origin or by acute phase mediators. We demonstrate herein that apart from its role in Hb catabolism, CD163 (i.e. membrane bound (mCD163) and/or soluble (sCD163) form) has immunoregulatory properties mediated in part through binding/interaction with a novel ligand (referred to herein as CD163-ligand) besides haptoglobin-hemoglobin complexes. For example sCD163 hampers proper immune responses against tumors or infections by dampening of the immune system mediated in part through its ligand (CD163-ligand). We demonstrate herein that CD163 (membrane bound and/or soluble/secreted/shed form) has an immunomodulatory effect on the antigen specific activation of T lymphocytes which can be mediated in part by binding either to a CD163-ligand present on the cell surface of antigen specific T lymphocytes or to its soluble form (sCD163-ligand).

Inflammation is a major hallmark of a wide range of diseases such as autoimmune diseases, inflammatory diseases, organ rejection, and infectious diseases. Inflammation is characterized by an influx of cells of the immune system, the release of cytokines and other inflammatory mediators such as histamine, leukotriens and prostaglandins, resulting in fever and tissue destruction. Pharmaceutical agents used to modulate inflammation in a host should ideally be endogenous substances, such as therapeutical proteins. These therapeutical proteins should not be recognized as foreign agents (i.e. no neutralizing antibodies will be formed, no nephrotoxicity is expected from endogenous proteins and no complexes are expected to be formed due to an antibody response against the therapeutical protein). Although many pharmaceutical compounds are currently used to treat inflammatory diseases, there is a need for more effective compounds (immunosuppressive molecules) with lowered toxicity profiles.

Currently used pharmacological therapies in immune related diseases in many cases only provide temporal relief. In addition drugs used to combat inflammation are not very selective, targeting non-inflammatory cells as well as inflammatory cells and often have moderate to serious side effects after chronic treatment, especially in children. Many patients become resistant to the drugs (e.g. glucocorticoid) used and high doses are associated with unpleasant side effects. Hence, there is a strong need for safer, more selective and more efficacious therapies which display a long-term clinical benefit to patients suffering from immune related disorders.

A more fundamental treatment aimed at antigen-specific T-lymphocytes and antigen-presenting cells is desirable since these cell-types play a crucial role in the initiation and progression of immune disorders. Immune related disorders often characterised by inflammation may be due to either immune heightening such as in the case of auto-immune diseases or immune dampening such as in the case of infection due to infectious agents. The present invention through the identification of a ligand for CD163 (membrane bound and/or soluble/secreted/shed form) provides an efficacious method to modulate undesirable immune response mediated through CD163 or its ligand CD163-ligand.

The invention provides a method for identifying a molecule with immune modulatory activity capable of interacting with CD163 comprising providing CD163 or a functional part, derivative and/or analogue thereof and under suitable conditions detecting a molecule capable of interacting with said CD163 and determining whether said molecule is capable of modulating an immune response. CD163 as used herein refers to the membrane bound and/or its soluble (i.e. secreted/shed) form. For instance, said CD163 is a receptor or functional fragment thereof present on cells of myeloid lineage (monocytes, macrophages, dendritic cells) and/or cells of lymphoid lineage and its soluble form (sCD163) is the shed product of this receptor. A molecule as used herein can be any substance be it nucleic acid, amino acid, a carbohydrate or a lipid comprising moiety (or combinations thereof), or any other moiety that can interact with CD163 and as a result of said interaction an immune response is modulated (i.e. instigated or suppressed). It is understood that said molecule with immune modulatory activity can be cell/membrane bound and/or soluble. It is also understood that said molecule can block the active site of CD163 and in doing so can modulate an immune response. The active site as used herein is the binding/adherence site on CD163 for a particular substance (e.g. a functional site such as a receptor-binding cavity). In a preferred embodiment of a method of the invention said molecule with immune modulatory activity is present on cells of lymphoid lineage (e.g. T cells, B cells), or endothelium and comprises a CD163-ligand. For example a CD163-ligand (e.g. sCD163-ligand) can be used to neutralise CD163 (e.g. sCD163) in situations where CD163 is increased. This can at least in part reduce the immunosuppressive effects of CD163 (e.g. sCD163), and serve to stimulate an immune response. On the other hand a monovalent CD163-ligand can bind to membrane bound CD163 (mCD163) preventing the activation of CD163 bearing cells, serving to suppress an immune response.

An immune response is a physiological response of an organism to agents (e.g. infectious agents, proteins, (tumor) cells etc.) that pose a threat to said organism. For example an immune response can involve activation and production of factors by leucocytes cells comprising B lymphocytes and T lymphocytes, NK cells, granuloctes, monocytes, macrophages and dendritic cells. Two types of immune responses are well recognized, namely the innate immune response and the adaptive immune response. An adaptive immune response is highly antigen-specific and can generate long lived immune memory. Cells involved in the adaptive immune response (henceforth called antigen-specific immune response) such as B lymphocytes and T lymphocytes recognize their antigens through highly specific cell surface receptors. Innate immune responses are responses involving granulocytes and monoctes, macrophages and dendritic cells and NK cells. These cells recognize frequently encountered antigens with germline encoded receptors (Pattern Recognition Receptors (PRR)), or the constant region of antibodies, thus providing a first line of defense before the acquired immune system is able to mount a response. PRR receptors when they bind their ligand transmit signals into the immune cell which can lead to the release of biological mediators that can instruct the adaptive (acquired) as well as innate arm of the immune response.

Modulation as used herein can refer to up-regulation or down-regulation of an immune response, for example by activation and/or suppression of gene(s) which are essentially capable of initiation and/or progression and/or suppression and/or repression of an immune response and/or symptoms of said immune response. Said modulation can be mediated by positive (i.e. up-regulation) or negative (i.e. down-regulation) regulation of gene transcription, and/or by the modification of a gene and/or gene product (e.g. post-translational modification). A functional part of CD163 is defined as a part of CD163 which has the same properties as CD163 in kind but not necessarily in amount. A functional derivative of CD163 is defined as CD163 which has been altered such that the properties of the altered CD163 are essentially the same in kind, but not necessarily in amount. Suitable derivatives can be generated through using codon degeneracy, for example by conservative amino acid substitution. A functional analogue of CD163 is a homologue and/or functional equivalent of CD163 which can be derived from a different species and/or generated synthetically.

In a preferred embodiment of a method of the invention said CD163 comprises a soluble CD163. A soluble CD163 (sCD163) is a secreted or shed form of the membrane bound CD163. In another preferred embodiment of a method of the invention said molecule with immune modulatory activity comprises a proteinaceous molecule, functional derivative, functional fragment and/or analogue thereof. A proteinaceous molecule as used herein can be any amino acid comprising moiety. In one embodiment said proteinaceous molecule comprises a CD163 ligand. A CD163 ligand is a proteinaceous molecule encoded by a nucleic acid of a cell. A functional part of said CD163 ligand is defined as a part of a CD163 ligand which has the same immunomodulatory properties in kind as a CD163 ligand but not necessarily in amount. By immunomodulatory properties is meant the capability to induce or inhibit an immune response in a host cell. A functional derivative of a CD163 ligand is defined as a CD163 ligand which has been altered such that the properties (e.g. immunomodulatory properties) of said altered CD163 ligand are essentially the same in kind, but not necessarily in amount. Suitable derivatives can be generated through using codon degeneracy, for example by conservative amino acid substitution. A functional analogue of said CD163 ligand is a homologue and/or functional derivative (i.e. functional equivalent) of said CD163 ligand which can be derived from a different species and/or generated synthetically. As used herein, the term "functional equivalent" means that the amino acid of a proteinaceous molecule according to the invention can be modified by means of one or more substitutions, deletions, or additions, the net effect of which does not result in a functional dissimilarity in kind not necessarily in amount between the amino acid of a proteinaceous molecule according to the invention and the modified form.

In a preferred embodiment the invention provides an isolated and/or recombinant and/or synthetic molecule obtainable by a method according to the invention. In one aspect the invention provides a substantially isolated or purified CD163 ligand or a recombinant (i.e. a modified form generated through genetic engineering approaches) or a synthetic (i.e. artificially generated as opposed to naturally occurring, for example by chemical synthesis) form thereof having substantially similar immunomodulatory activity. As used herein, the term "substantially isolated or purified" refers to a molecule according to the invention that is removed from its natural environment, isolated or separated, and is essentially free from components with which it is naturally associated. In terms of the invention the phrase "substantially similar immunomodulatory activity" means that said natural, recombinant or synthetic CD163-ligand polypeptide according to the invention, or any oligopeptide thereof, is similarly immunologically active in kind not necessarily in amount, that is it is capable of inducing a specific immune response in a mammal or a cell. Preferably said molecule is of mammalian or avian origin. It is understood that said CD163- ligand can be cell/membrane bound (e.g. a cell surface receptor, for example on cells of lymphoid lineage, endothelial cells and/or myeloid lineage or soluble (e.g. freely circulating). For instance the soluble form of the CD163- ligand can arise as a result of shedding of the membrane bound form. Preferably said CD163-ligand soluble form (sCD163-ligand) comprises at least part of the extracellular and/or cytoplasmic domain, more preferably the CD163-binding domain of a cell/membrane bound CD163-ligand (mCD163-ligand). An antigen presenting cell (APC) can be any cell (e.g. macrophages, endothelium, dendritic cells, langerhans cells of the skin etc.) which carries on its surface antigen bound to major histocompatibility complex (e.g. MHC class I or Class II) molecules and presents the antigen in this context to cells of the immune system. It is also understood that a molecule of the present invention can inhibit or induce signal transduction into antigen presenting cell (APC). Single binding of said molecule to CD163 can result in inhibition of signal transduction into antigen presenting cells (e.g. macrophages) whereas cross-linking/ multiple binding of said molecule to CD163 can serve to induce signal transduction into antigen presenting cells. Thus a molecule of the invention can inhibit or induce signal transduction into antigen presenting cell (APC) depending on the valency of binding, that is to say mono-valent binding of said molecule to CD163 can result in inhibition of signal transduction into antigen presenting cells, whilst muti-valent binding can induce signal transduction, or vice versa.

In another preferred embodiment the invention provides a molecule obtainable by a method according to the invention. Preferably said molecule comprises a receptor or functional fragment thereof. For instance, said CD163-ligand can be a receptor or functional part thereof present on cells of lymphoid lineage and/or endothelial cells and/or cells of myeloid lineage (monocytes, macrophages, dendritic cells) and its soluble form (sCD163-ligand) is the shed product of this receptor, which may be capable of freely circulating in body fluids. A receptor is a molecular structure within a cell or on the surface characterised by selective binding of a specific substance and a specific physiologic effect that accompanies the binding (e.g. cell surface receptors or intracellular receptors). A functional fragment of a receptor is any part of the receptor involved in the recognition of a substance wherein said recognition involves activation and/or inactivation of the receptor.

In another aspect the invention provides a molecule according to the invention coupled to a moiety. For instance a molecule or functional fragment thereof, such as a proteinaceous molecule of the invention can be linked to a second or subsequent moiety to form a fusion protein. In terms of the invention said moiety can serve to confer additional properties to the proteinaceous molecule (e.g. increased longevity and stability, improved intracellular targeting) or to improve its biological activity (i.e. imunomodulatory activity and/or pharmacokinetics). Suitable moieties can include a molecule comprising at least part of an immunoglobulin chain (e.g. a constant region of said chain), a molecule with immunoregulatory activity like a cytokine (e.g. IL-10, 1L-12, GSCF, IFN-γ, etc.), a toxic moiety, and a protection molecule like polyethylene glycol (PEG). It is understood that by using a nucleic acid encoding a molecule, or a fragment thereof, of the present invention any combination of fusion protein(s) can also be generated using recombinant techniques known in the art. For example a fusion protein of CD163-ligand (e.g. sCD163-ligand) coupled to the constant region of an immunoglobulin (e.g. sCD163-ligand-Fc) can be used to neutralise CD163 (e.g. sCD163) and/or to cross link membrane bound CD163 to induce activation of CD163 bearing cells. This can at least in part reduce the immunosuppressive effects of CD163 (e.g. sCD163), and serve to stimulate an immune response. Such a fusion protein has an advantage over a monovalent sCD163-ligand alone in that it has a longer life in circulation and has an additive effect on augmenting an immune response.

In another aspect the invention provides use of a molecule according to the invention to modulate an immune response. Said immune response can comprise an innate and/or an antigen specific (adaptive) immune response.

Preferably said immune response comprises an antigen specific (adaptive / acquired immune) response.

In inflammatory diseases such as autoimmune diseases, allergy, asthma, transplant rejection the activation of the immune system is either unwanted or excessive. These diseases are highly specific for the antigens that are recognized by the immune system such as auto-antigens (autoimmune diseases), allo-antigens (transplant rejection), allergens (allergy) or infectious agents (certain infections). In the case of an immune response which comprises an inflammatory response a molecule according to the invention can be used to inhibit T-cell responses and/or migration of CD163 expressing cells over endothelium. In diseases, such as monomyeloid leukemias and severe infection serum levels of sCD163 are increased which causes immune damping and as a result there is an insufficient immune response to the leukemia or infectious agent.

Herein we disclose that interaction of CD163-ligand with membrane bound CD163 can induce activation and cytokine production of the cell bearing CD163 (e.g. an antigen presenting cell, like a macrophage). The presence in serum of sCD163 may be the natural method to inhibit CD163-CD163-ligand interaction by engaging the CD163-ligand (both soluble and membrane bound form). This prevents activation of CD163 bearing cells and can serve to deliver an inhibitory signal to cell types expressing the CD163-ligand, like T cells thus promoting immune dampening.

In yet another aspect the invention provides use of a molecule and/or functional derivative and/or functional fragment and/or analogue thereof according to the invention for modulating an immune response wherein said modulation comprises augmentation of an immune response. As a way of illustration but not as a way of limitation by modulating the activity of CD163 and/or a CD163-ligand of the present invention (such as soluble CD163-ligand), one is capable of stimulating an immune response in cancer or infectious diseases in which serum soluble CD163 levels are increased (i.e. by shedding of the membrane bound form). This can be achieved in a number of ways.

One way to stimulate an immune response is by neutralizing soluble CD163-induced immune dampening. For example by providing an excess of sCD163-ligand, and/or an excess of sCD163-ligand coupled to a moiety as mentioned previously, and/or an excess of an antagonist II of CD163, one can neutralise the dampening effects of CD163. The sCD163-ligand can engage the sCD163 shed from cell membranes under disease conditions, and this can effectively neutralise the inhibitory signal induced by sCD163 binding the cell bound CD163-ligand on T cells or other CD163-ligand bearing cells. Also with an antagonist II of CD163 of the present invention, it is now for instance possible to neutralise CD163 (e.g. sCD163). By neutralizing CD163, the binding/interaction of a molecule of the invention (e.g. CD163-ligand) with CD163 (e.g. sCD163) is at least in part reduced. Neutralizing CD163 can serve to counteract the dampening effects of CD163 on immune responses, like T-cell responses.

Another way to stimulate an immune response is to cross-link CD163 on CD163-bearing cells (e.g. antigen presenting cells like macrophages) through the administration of a bivalent or multivalant sCD163-ligand or an agonistic II antibody to CD163. A bivalent sCD163-ligand can comprise an Ig-fusion protein in which two sCD163-ligand molecules are constructed on one Ig-backbone. A multivalent sCD163-ligand can be prepared for example using multiple sCD163-ligand molecules on a solid phase or high affinity tags that can be cross-linked like (strept)avidin-biotin. This will serve to induce signal transduction into CD163-bearing cells (e.g. antigen presenting cells like macrophages) resulting in stimulation of the CD163-bearing cells. Furthermore, since the CD163-ligand is the natural ligand for CD163, this means that its modulation within the context of the present invention, will not provoke severe side-effects and harmful immune responses in an organism.

In another aspect the invention provides use of a molecule and/or functional derivative and/or functional fragment and/or analogue thereof according to the invention for modulating an immune response wherein said modulation comprises suppression of an immune response. As a way of illustration but not as a way of limitation by modulating the expression of a CD163-ligand of the present invention (such as soluble CD163-ligand), one is capable of suppressing an immune response in auto-immune disease like rheumatoid arthritis, diabetes, multiple sclerosis, systemic lupus erythematosous, psoriasis, autoimmune thyroidits, inflammatory diseases, transplantation diseases, infectious diseases (i.e. septic shock), etc.

It is understood that a molecule with immune modulatory activity according to the invention (e.g. sCD163-ligand) can induce or prevent signal transduction into CD163-bearing cells, like macrophages. Interaction of a membrane bound and/or soluble molecule of the invention with membrane bound CD163 induces activation and cytokine production of a cell bearing CD163 (e.g. an antigen presenting cell, like a macrophage). This may or may not depend on the valency of the binding. Said molecule of the invention (e.g. sCD163-ligand) or an antagonist II to CD163 of the invention is particularly suitable for the inhibition of immune responses in inflammatory diseases in which serum sCD163 levels are not increased. Monovalent binding can result in inhibition of signal transduction into macrophages, whilst cross-linking can induce signal transduction. Said molecule with immune modulatory activity according to the invention (e.g. sCD163-ligand) can bind in monomeric fashion to CD163 preventing cross-linking of CD163 on CD163-bearing cells and can thus prevent efficient activation of antigen presenting cells (e.g. monocytes and/or macrophages) through CD163.

In another aspect the invention provides an antagonist of a molecule according to the invention. Said antagonist, further referred to as antagonist I, is a substance that at least in part tends to nullify the action of a molecule of the invention, whether said molecule is membrane bound or soluble. Preferably said antagonist I is an antibody or a functional part, derivative and/or analogue thereof. A functional part of an antibody is defined as a part which has the same kind of binding properties in kind, not necessarily in amount (e.g. a FAB fragment). A functional derivative of an antibody is defined as an antibody which has been altered such that the binding properties of said antibody are essentially the same in kind, not necessarily in amount. A derivative can be provided in many ways, for instance through conservative amino acid substitution. A non-limiting example of a suitable derivative is a single chain antibody. A non-limiting example of a suitable analogue is a synthetic antibody selected from a recombinant antibody library. In a preferred embodiment an antibody of the invention is a monoclonal antibody or a functional part, derivative and/or analogue thereof. A monoclonal antibody of the invention can be generated by standard immunization and cell fusion techniques or by combinatorial library approaches. It is also understood that said antagonist I of the present invention can comprise a chimeric antibody, a humanized antibody or a fully human antibody. It is also understood that said antagonist I can comprise an antibody with a neutralising and/or blocking function.

The invention further provides an antagonist I coupled to a moiety. In terms of the invention said moiety can serve to confer additional properties to said antagonist I (e.g. increased longevity and stability, improved intracellular targeting) or to improve its biological activity (i.e. imunomodulatory activity and/or pharmacokinetics). Suitable moieties can include a molecule comprising at least part of an immunoglobulin chain (e.g. a constant region of said chain), a molecule with immunoregulatory activity like a cytokine (e.g. IL-10, 1L-12, GSCF, IFN-γ, etc.), a toxic moiety, and a protection molecule like polyethylene glycol (PEG). It is understood that by using a nucleic acid encoding an antagonist I, or a fragment thereof, any combination of fusion protein(s) can also be generated using recombinant techniques known in the art.

The invention as disclosed herein thus provides a method(s) using antibodies of an isolated and/or recombinant and/or synthetic proteinaceous molecule according to the invention to induce or prevent signalling through CD-163 and/or CD-163 ligand. An antibody of the present invention can bind to mCD163-ligand and/or sCD163-ligand and can either prevent signalling through the CD163-ligand or can induce or enhance signalling through the CD163 ligand. Thus an antibody of the present invention can have immunomodulatory effects, either by antagonistic or agonistic effects on signalling through the CD163-ligand. It is understood that an antibody of the present invention can bind to mCD163-ligand and/or sCD163-ligand and have no immunomodulatory activity (i.e. will not influence signalling through the CD163-ligand). It is also understood that an antibody of the present invention can induce the mCD163-ligand to be internalised or shed from the membrane. Moreover an antibody of the present invention can be coupled to an effector molecule (e.g. a cytokine) to increase the immunomodulatory capacity of said antibody. Additionally an antibody of the present invention can be coupled to a toxic moiety, allowing the reduction or a depletion of cells expressing the CD163-ligand.

In another aspect the invention provides use of an antagonist I to modulate an immune response. An antagonist I CD163 ligand-specific antibody of the present invention can bind the CD163-ligand on CD163-ligand bearing cells and can neutralise the CD163-induced in-activation of these cells. Furthermore, an antagonist I CD163 ligand-specific antibody of the present invention can prevent or overcome the signalling that is induced by signal transduction through the CD163-ligand, or prevent the interaction of CD163-ligand with CD163 or sCD163. This can counteract (i.e. at least in part nullify) the CD163 ligand mediated dampening of immune responses. Also an antagonist I CD163 ligand-specific antibody of the present invention can induce shedding or endocytosis of CD163-ligand, thus decreasing CD163-ligand expression on CD163-ligand bearing cells, hence serving to induce an immune response.

Therefore an antagonist I can be used to modulate an immune response, preferably in a CD163 related pathway, wherein said modulation comprises augmentation (i.e. amplification) of an immune response. With an antagonist I (e.g. CD163 ligand-specific antibody) of the present invention, and/or an antagonist II (e.g. sCD163-specific antibody) of the invention, and/or a molecule according to the invention (e.g. CD163-ligand, like sCD163-ligand), it is now for instance possible to efficiently block the immune dampening effect of sCD163 in diseases where elevated serum levels of sCD163 are observed. This serves to boost an organisms immune response against tumor cells or where there is an insufficient response against infectious pathogens. In this case, the membrane bound form of the CD163-ligand (mCD163-ligand) is not necessarily prevented from binding to CD163 or sCD163, and is therefore able to stimulate CD163 expressing cells such as macrophages, resulting in fewer persistent tumours and/or infections. Furthermore, antagonistic antibodies of a CD163-ligand mediate direct activation of CD163-ligand expressing immune cells like T cells, resulting in fewer persistent tumours or infections.

The invention also provides an agonist of a molecule according to the invention. Said agonist, further referred to as agonist I, is a substance that tends to at least in part trigger/stimulate the action of a molecule of the invention (e.g. an agonist can trigger/stimulate the immunosuppressive function of a molecule of the invention). Preferably said agonist I is an antibody or a functional part, derivative and/or analogue thereof. A functional part of an antibody is defined as a part which has the same kind of binding properties in kind, not necessarily in amount (e.g. a FAB fragment). A functional derivative of an antibody is defined as an antibody which has been altered such that the binding properties of said antibody are essentially the same in kind, not necessarily in amount. A derivative can be provided in many ways, for instance through conservative amino acid substitution. A non-limiting example of a suitable derivative is a single chain antibody. A non-limiting example of a suitable analogue is a synthetic antibody selected from a recombinant antibody library. In a preferred embodiment an antibody of the invention is a monoclonal antibody or a functional part, derivative and/or analogue thereof. A monoclonal antibody of the invention can be generated by standard immunization and cell fusion techniques or by combinatorial library approaches. It is also understood that said agonist I can comprise a chimeric antibody, a humanized antibody or a fully human antibody.

An agonist I can bind a molecule of the invention, for instance mimicking the effects of binding of sCD163 to a molecule of the invention. It is understood that an agonist I can bind with a higher avidity to a molecule of the invention. This can give rise to at least a similar or even a superior induction of an immunosuppressive signal through a CD163-ligand (e.g. membrane bound).

In another aspect the invention provides an agonist I coupled to a moiety. For instance linked to a second or subsequent moiety to form a fusion protein. In terms of the invention said moiety can serve to confer additional properties to the agonist I (e.g. increased longevity and stability, improved intracellular targeting) or to improve its biological activity (i.e. immunomodulatory activity and/or pharmacokinetics). Suitable moieties can include a molecule comprising at least part of an immunoglobulin chain (e.g. a constant region of said chain), a molecule with immunoregulatory activity like a cytokine (e.g. IL-10, 1L-12, GSCF, IFN-γ, etc.), a toxic moiety, and a protection molecule like polyethylene glycol (PEG). It is understood that by using a nucleic acid encoding an agonist I, or a fragment thereof, any combination of fusion protein(s) can also be generated using recombinant techniques known in the art.

Additionally the invention provides use of an agonist I to modulate an immune response. Preferably said modulation comprises suppression of an immune response. It is understood that an agonist I through interacting with a CD163-ligand can serve to inhibit an immune response, for instance an antigen specific (adaptive) immune response. An agonist I can by itself or in association with CD163 deliver a signal (e.g. inhibitory signal) through the CD163-ligand on CD163 ligand bearing cells, and this can inactivate the immune system. Another way to inhibit an immune response is to cross-link CD163-ligand on CD163-ligand bearing cells (e.g. T-cells) through the administration of an agonist I and/or sCD163, preferably through the administration of a bivalent and/or multivalent agonist I and/or bivalent and/or multivalent sCD163 (e.g. CD163 coupled to a constant region of an immunoglobulin). A bivalent sCD163 can comprise an Ig-fusion protein in which two sCD163 molecules are constructed on one Ig-backbone. A multivalent sCD163 can be prepared for example using multiple sCD163 molecules on a solid phase or high affinity tags that can be cross-linked like (strept)avidin-biotin. This will serve to induce signal transduction into CD163-ligand bearing cells (e.g. T-cells) resulting in stimulation of the CD163-ligand bearing cells.

An agonist I of a molecule of the invention can for instance enhance sCD163 binding to the CD163-ligand and/or increase the residence time of sCD163 bound to the CD163-ligand which serves to increase signal transduction through the CD163-ligand on CD163-ligand bearing cells and thus inactivate the immune system. Agonist I antibodies of a CD163-ligand can deliver a signal (e.g. inhibitory signal) by a synergistic interaction with sCD163, thereby enhancing the signal induced by sCD163 thereby inhibiting an immune response. This would be particularly efficacious for the prophylactic or therapeutic treatment of immunological diseases, such as for instance autoimmune diseases, inflammatory diseases, transplant rejection, or infectious diseases. Such an agonist I can at least in part prevent further activation of CD163-ligand bearing cells, such as T cells or other cells of the immune system. Furthermore such agonist I can at least in part prevent or overcome endocytose or shedding of CD163-ligand from CD163 ligand bearing cells thereby increasing the net amount of CD163-ligand present on CD163-ligand bearing cells.

For instance, an agonist I can upon interaction (e.g. binding) with a molecule of the invention deliver a signal (e.g. an inhibitory/immunosuppressive signal) to cell types expressing a molecule of the invention, like T cells, resulting in dampening of an immune response. Preferably said immune response comprises an antigen specific (adaptive immune) response. In the case of an immune response which comprises an inflammatory response, an agonist I, can be used to inhibit T-cell responses and/or migration of CD163-expressing cells over endothelium.

The invention further provides an isolated and/or recombinant and/or synthetic CD163, a functional part, derivative and/or analogue thereof. Preferably said CD163 comprises a soluble CD163. In a preferred embodiment of the invention said soluble CD163 comprises a C-terminus and/or N-terminus identical to the natural or PMA- induced shed CD163 molecule. Preferably said N-terminal end comprises APGWANS, and said C-terminus comprises GPIWLNEVK. The invention provides a substantially isolated or purified CD163 or a recombinant (i.e. a modified form generated through genetic engineering approaches) or a synthetic (i.e. artificially generated as opposed to naturally occurring, for example by chemical synthesis) form thereof having substantially similar immunomodulatory activity. As used herein, the term "substantially isolated or purified" refers to CD163 according to the invention removed from its natural environment, isolated or separated, and is essentially free from components with which it is naturally associated. In terms of the invention the phrase "substantially similar immunomodulatory activity" means that said natural, recombinant or synthetic CD163 polypeptide according to the invention, or any oligopeptide thereof, is similarly immunologically active in kind not necessarily in amount. A functional part of CD163 is defined as a part of CD163 which has the same properties as CD163 in kind but not necessarily in amount. A functional derivative of CD163 is defined as CD163 which has been altered such that the properties of the altered CD163 are essentially the same in kind, but not necessarily in amount. Suitable derivatives can be generated through using codon degeneracy, for example by conservative amino acid substitution. A functional analogue of CD 163 is a homologue and/or functional equivalent of CD163 which may be derived from a different species and/or generated synthetically.

The invention further provides an isolated CD163 coupled to a moiety. For instance an isolated CD163, a functional part, derivative and/or analogue thereof according to the invention can be linked to a second or subsequent moiety to form a fusion protein. In terms of the invention said moiety can serve to confer additional properties to said isolated CD163 molecule (e.g. increased longevity and stability, improved intracellular targeting) or to improve its biological activity (i.e. imunomodulatory activity and/or pharmacokinetics). Suitable moieties can include a molecule comprising at least part of an immunoglobulin chain (e.g. a constant region of said chain), a molecule with immunoregulatory activity like a cytokine (e.g. IL-10, 1L-12, GSCF, IFN-γ, etc.), a toxic moiety, and a protection molecule like polyethylene glycol (PEG). In a preferred embodiment of the present invention said moiety comprises a constant region of an immunoglobulin.

In another aspect the invention provides an antagonist of CD163. An antagonist, further referred to as antagonist II of CD163, can in part neutralise CD163 (e.g. sCD163). By neutralizing CD163, the binding/interaction of a molecule of the invention (e.g. CD163-ligand) with CD163 (e.g. sCD163) is at least in part reduced. Neutralizing CD163 can serve to counteract the dampening effects of CD163 on immune responses, like T-cell responses. For instance an antagonist II can serve to augment an immune response through neutralisation of sCD163, by preventing the interaction of CD163 with the CD163 ligand.
Preferably said antagonist II is an antibody or functional part, derivative and/or analogue thereof. It is also understood that said antagonist can comprise an antibody with a neutralising and/or blocking function.

The invention also provides an antagonist II coupled to a moiety. In terms of the invention said moiety can serve to confer additional properties to said antagonist II of CD163 (e.g. increased longevity and stability, improved intracellular targeting) or to improve its biological activity (i.e. imunomodulatory activity and/or pharmacokinetics). Suitable moieties can include a molecule comprising at least part of an immunoglobulin chain (e.g. a constant region of said chain), a molecule with immunoregulatory activity like a cytokine (e.g. IL-10, 1L-12, GSCF, IFN-γ, etc.), a toxic moiety, and a protection molecule like polyethylene glycol (PEG). For example, said antagonist II can comprise an antibody which can be coupled to a toxic moiety, allowing the reduction or a depletion of cells expressing CD163. In the same manner a toxic moiety can also be coupled to sCD163 or a fragment thereof, to reduce or eliminate its target cells.

The invention further provides an agonist of CD163, further referred to as agonist II. Preferably said agonist II is an antibody or functional part, derivative and/or analogue thereof. It is understood that said agonist II can serve to augment an immune in a similar manner to an antagonist II of CD163, through its ability to neutralise sCD163. The invention further provides an agonist II coupled to a moiety. In terms of the invention said moiety can serve to confer additional properties to said agonist II of CD163 (e.g. increased longevity and stability, improved intracellular targeting) or to improve its biological activity (i.e. imunomodulatory activity and/or pharmacokinetics). Suitable moieties can include a molecule comprising at least part of an immunoglobulin chain (e.g. a constant region of said chain), a molecule with immunoregulatory activity like a cytokine (e.g. IL-10, 1L-12, GSCF, IFN-γ, etc.), a toxic moiety, and a protection molecule like polyethylene glycol (PEG). One way to stimulate an immune response is by neutralizing soluble CD163-induced immune dampening. For example by providing an excess of sCD163-ligand, and/or an excess of sCD163-ligand coupled to a moiety as mentioned previously, and/or an excess of an antagonist II of CD163 and/or an excess of an agonist II of CD163, and/or an excess of an antagonist II of CD163 coupled to a moiety and/or an excess of an agonist II of CD163 coupled to a moiety, one can neutralise the dampening effects of CD163. The antagonist II of CD163 and/or agonist II of CD163 can engage the sCD163 shed from cell membranes under disease conditions, and this can effectively neutralise the inhibitory signal induced by sCD163 binding the cell bound CD163-ligand on T cells or other CD163-ligand bearing cells. Neutralizing CD163 can serve to counteract the dampening effects of CD163 on immune responses, like T-cell responses.

Furthermore the invention provides a method to detect the presence of a molecule according to the invention in a sample comprising contacting the sample with a binding molecule for said molecule according to the invention to form a complex, further comprising detecting said complex in the sample. For example said binding molecule is any entity, be it nucleic acid, amino acid, a carbohydrate or a lipid comprising moiety (or combinations thereof), than can bind a molecule of the invention. Preferably said entity is coupled/linked to a moiety which enables the detection of a molecule of the invention in a sample.

Preferably said binding molecule comprises an antagonist I or agonist I according to the invention. For example said antagonist I or agonist I can have a moiety attached, which can be for example recognised by a detection/determining substance (e.g. a label). For instance a visually detectable or direct label (e.g. radioactive label, enzyme label, fluorescent label chemiluminescent label, bioluminescent label, gold label etc.). Examples of commonly used enzyme labels are horseradish peroxidase, alkaline phosphatase and β-galactosidase etc. Also preferred is that said binding molecule comprises membrane bound CD163 and/or sCD163.

In yet another aspect the invention provides a method to determine the binding activity of a molecule according to the invention in a sample comprising detecting the presence of a molecule using a method according the invention, further determining the levels(i.e. amount) of binding molecule-molecule (i.e. complex) in the sample. It is therefore an object of the present invention to monitor an immune response comprising detecting the presence of and/or monitoring the binding activity of a molecule in a sample. Screening technologies are known in the art for example proteomic technologies.

The invention provides a method for the production of a diagnostic kit comprising a method to detect the presence of a molecule according to the invention in a sample and/or to determine the binding activity of a molecule according to the invention in a sample. Suitable basis for a diagnostic kit are known in the art.

In one aspect the invention provides a nucleic acid, functional part, functional derivative and/or analogue thereof encoding a molecule according to the invention. A functional part of a nucleic acid of the invention is a part of said nucleic acid whose encoded product is capable of modulating an immune response, preferably in a CD163 related pathway, in an organism. A functional derivative of a nucleic acid of the invention is any nucleic acid produced from or related to said nucleic acid, which retains the same properties as said nucleic acid in kind not necessarily in amount. An analogue of a nucleic acid of the invention can be for example an allelic variant.

In another aspect the invention provides a nucleic acid functional part, functional derivative and/or analogue thereof encoding an antagonist I of a molecule according to the invention. In another aspect the invention provides a nucleic acid, functional part, functional derivative and/or analogue thereof encoding an agonist I of a molecule according to the invention. In yet another aspect the invention provides a nucleic acid encoding an isolated CD163 according to the invention. In yet another aspect the invention provides a nucleic acid encoding an antagonist II or an agonist II of an isolated CD163 according to the invention.

In yet another aspect the invention provides a vector comprising a nucleic acid according to the invention. Suitable vectors are known to one of skill in the art, for example plasmid vectors, viral vectors etc. In another aspect the invention provides a cell comprising a vector according to the invention. Preferably a mammalian or avian cell. Furthermore the invention provides a gene delivery vehicle comprising a vector according to the invention. A gene delivery vehicle as used herein is any vehicle that can deliver a nucleic acid of the invention to an organism, for the purpose of modulating an immune response in an organism. The invention also provides the use of a gene delivery vehicle of the invention for the preparation of a medicament.

The invention provides a method for the production of a molecule according to the invention, or an antagonist I of a molecule according to the invention or an agonist I of a molecule according to the invention, or CD163 according to the invention, or an antagonist II of CD163 according to the invention or an agonist II of CD163 according to the invention in an organism comprising inserting into the genome of said organism one or more copies of a nucleic acid according to the invention. An organism in the context of the present invention can be for example a micro-organism (e.g. Archaea, Bacteria, Cyanobacteria, Microalgae, Fungi, Yeast, Viruses, Protozoa, Rotifers, Nematodes, Micro-Crustaceans, Micro-Molluscs, Micro-Shellfish, Micro-insects etc.), a plant, a non-human animal and a plant or animal cell (e.g. artificial cell, cell culture or protoplast etc.).

In yet another aspect the invention provides use of an isolated CD163 according to the invention and/or an antagonist II of CD163 according to the invention and/or an agonist II of CD163 according to the invention to modulate an immune response. For instance, said CD163 according to the invention can be linked to a second or subsequent moiety to form a fusion protein. In terms of the invention said moiety can serve to confer additional properties to said CD163 molecule (e.g. increased longevity and stability, improved intracellular targeting) or to improve its biological activity (i.e. imunomodulatory activity and/or pharmacokinetics). Suitable moieties can include a molecule comprising at least part of an immunoglobulin chain (e.g. a constant region of said chain), a molecule with immunoregulatory activity like a cytokine (e.g. IL-10, 1L-12, GSCF, IFN-γ, etc.), a toxic moiety, and a protection molecule like polyethylene glycol (PEG).

For example, the use of a fusion protein of CD163 (e.g. sCD163) coupled to the constant region of an immunoglobulin (e.g. CD163-Fc) can have several advantages over unmodified CD163. For instance, a CD163-Fc has an increased half life in circulation. Additionally a CD163-Fc can be generated as a monovalent (i.e. one CD163 coupled to the constant region of an immunoglobulin chain) molecule, or more preferred as a bivalent (i.e. one or two CD163 moieties coupled to the constant region of an immunoglobulin chain) molecule. A bivalent CD163-Fc molecule is likely to crosslink a molecule of the invention (e.g. CD163-ligand), thus generating an immunosuppressive signal, and because of its bivalency, the molecule can bind with a higher avidity to a molecule of the invention.

For instance, an isolated CD163 according to the invention and/or an isolated CD163 coupled to a moiety, can upon binding to a molecule of the invention, preferably a membrane bound molecule, deliver a signal (e.g. inhibitory signal) to cell types expressing a molecule of the invention resulting in dampening/suppression/inhibition of an immune response. Said CD163 and/or CD163 coupled to a moiety can for instance bind monovalently, bivalently and/or multivalently to a molecule according to the invention (i.e. CD163-ligand) and induce signal transduction through the CD163-ligand on CD163-ligand bearing cells and thus inactivate the immune system (e.g. dampen an immune response). Preferably said immune response comprises an antigen specific (adaptive immune) response. In the case of an immune response which comprises an inflammatory response CD163 and/or CD163 coupled to a moiety can be used to inhibit T-cell responses and/or migration of CD163-expressing cells over endothelium.

An antagonist II according to the invention is capable of binding to CD163 (e.g. sCD163) and of at least in part neutralizing the immune dampening effect of CD163 (e.g. sCD163). For instance, said antagonist II of CD163 can be an antibody, or a functional derivative thereof. An antagonist II of CD163 of the present invention is particularly suitable for inducing/stimulating/augmenting an immune response. An antagonist II of CD163 can prevent CD163 (e.g. sCD163) from binding to a molecule of the invention thereby preventing the delivery of a signal (e.g. inhibitory signal) though said molecule. An antagonist II of CD163 of the present invention is particularly suitable for induction of immune response in an organism, (e.g. mammal), in which the immune system is not efficiently stimulated as a result of elevated serum levels of sCD163.

With an antagonist II, it is now for instance possible to efficiently decrease unwanted high levels of circulating sCD163 in patients, suffering from improper immune response against tumour cells and/or improper immune response against pathogens without harmful side-effects. The presence in serum of sCD163 is a natural method to inhibit immune responses and can lead to unwanted and uncontrolled tumor growth and also facilitate infections with pathogens through its damping effect on immune responses.

Also with an antagonist II, it is now for instance possible to neutralise CD163 (e.g. sCD163). Neutralizing CD163 improves the capability of a patient to induce T-cell responses. Also by neutralizing CD163, the binding/interaction of a molecule of the invention (e.g. CD163-ligand) with CD163 (e.g. sCD163) is at least in part reduced. As a consequence a molecule of the present invention is free to stimulate antigen presenting cells like macrophages, resulting in fewer persistent tumours or infections.

An agonist II is capable of at least interacting (e.g. binding) with CD163, inducing the activation of a cell bearing CD163, resulting in the production of cytokines and/or other factors. Further, by an agonist II of CD163 according to the invention binding to CD163, the interaction of CD163, with a molecule according to the invention can in part be inhibited, preventing the generation of a signal to cells bearing a molecule of the invention. Thus by activating CD163 bearing cells and/or by preventing the inhibition of cells expressing a molecule of the invention an immune response is increased.

In another aspect the invention provides use of an antagonist II of an isolated and/or recombinant and/or synthetic CD163 according to the invention and/or a molecule according to the invention and/or a molecule according to the invention coupled to a moiety and/or an antagonist I of the molecule of the invention to modulate an immune response. Said immune response can comprises an innate and/or an antigen-specific (adaptive) immune response. Preferably said immune response comprises an antigen-specific (adaptive) immune response. In the case of an immune response which comprises an insufficient immune response in a disease characterized by elevated serum levels of sCD163 an antagonist II of an isolated and/or recombinant and/or synthetic CD163 according to the invention and/or a molecule according to the invention and/or a molecule according to the invention coupled to a moiety and/or an antagonist I of the molecule of the invention can be used to inhibit and/or overcome the immune dampening effect of CD163.

In another aspect the invention provides a pharmaceutical composition comprising a molecule according to the invention and/or an antagonist I of a molecule according to the invention and/or an agonist I of a molecule according to the invention and/or an isolated CD163 according to the invention and/or an antagonist II of an isolated CD163 and/or an agonist II of an isolated CD163 and/or a cell according to the invention and/or a gene delivery vehicle according to the invention. Suitable basis for pharmaceutical compositions are known in the art. Pharmaceutically acceptable carriers are well known in the art and include, but are not limited to, saline, buffered saline, dextrose, water, glycerol, sterile isotonic aqueous buffer, and combinations thereof. One example of such an acceptable carrier is a physiologically balanced culture medium containing one or more stabilizing agents such as stabilized, hydrolyzed proteins, lactose, etc. The carrier is preferably sterile.

In yet another aspect the invention provides a pharmaceutical composition according to the invention for augmenting or suppressing an immune response. Depending on the use (i.e. to enhance or suppress an immune response) a therapeutic or a prophylactic effective amount of a second substance (i.e. anti-inflammatory/immunosuppressive substance like cyclosporin A, FK506, sulfasalazine, antihistamines, bronchodilators, leukotrien inhibitors, (gluco)-corticosteroids, anti-TNF-α antibodies, anti-CD40 antibodies, or immunostimulatory substances like type I interferon, GCSF, IFN-gamma or other cytokines, adjuvants to induce inflammation) can be added to said pharmaceutical composition. Said pharmaceutical composition can also be used in combination with various cancer treatments, or treatments for infectious diseases (e.g. antibacterial, antifungal or antiviral substances).

In another aspect the invention provides use of a molecule according to the invention and/or an antagonist I of a molecule according to the invention and/or an agonist I of a molecule according to the invention and/or an isolated CD163 according to the invention and/or an antagonist II of an isolated CD163 according to the invention and/or an agonist II of an isolated CD163 according to the invention and/or a cell according to the invention and/or a gene delivery vehicle according to the invention in the preparation of a pharmaceutical composition for the therapeutic or prophylactic treatment of a disease, treatable by modulating an immune response.

On one hand a molecule according to the invention and/or a molecule of the invention coupled to a moiety (e.g. the constant region of an immunoglobulin (e.g. sCD163-ligand-Fc)) and/or an antagonist I of CD163-ligand and/or an antagonist II of CD163 and/or an agonist II of CD163 can be used to prevent the interaction of CD163 with the CD163-ligand, an/or to cross link membrane bound CD163 to induce activation of CD163 bearing cells, in situations in which CD163 is increased (for example cancer, infections etc). This can at least in part reduce the immunosuppressive effects of CD163 and serve to stimulate an immune response. This is particularly useful for the therapeutic or prophylactic treatment of an individual with an immune response disorder, like cancer, infections and other disease characterised by increased levels of sCD163.

On the other hand a molecule of the invention and/or an agonist I of a molecule of the invention and/or an isolated and/or recombinant and/or synthetic CD163 according to the invention and/or said isolated and/or recombinant and/or synthetic CD163 coupled to a moiety (e.g. the constant region of an immunoglobulin (e.g. sCD163-Fc)) can serve to suppress an immune response. This is particularly useful for the therapeutic or prophylactic treatment of an individual with an immune response disorder, like inflammatory diseases, autoimmune diseases, transplantation, allergy, asthma etc.

In another aspect the invention provides use of a molecule according to the invention and/or an antagonist I of a molecule according to the invention and/or an agonist I of a molecule according to the invention and/or an isolated CD163 according to the invention and/or an antagonist II of an isolated CD163 according to the invention and/or an agonist II of an isolated CD163 according to the invention and/or a cell according to the invention and/or a gene delivery vehicle according to the invention in the preparation of a pharmaceutical composition, wherein said disease comprises an autoimmune disease (e.g. rheumatoid arthritis, etc.), Hepatitis, inflammatory disease (e.g. inflammatory bowel disease etc.) allergy, asthma, cancer, infectious diseases (e.g. sepsis etc.), transplantation related diseases, host versus graft related diseases, cardiovascular disease and/or neurological diseases. It is understood that all diseases associated with increased serum soluble CD163 levels are included. Diseases associated with elevated levels of circulating sCD163 can be characterised by an insufficient immune response, like cancers (e.g. monomyeloid leukemias) and infectious diseases.

The invention provides a method of treatment of a immune related disease comprising a molecule according to the invention and/or an antagonist I of a molecule according to the invention and/or an agonist I of a molecule according to the invention and/or an isolated CD163 according to the invention and/or an antagonist II of an isolated CD 163 according to the invention and/or an agonist II of an isolated CD163 according to the invention and/or a cell according to the invention and/or a gene delivery vehicle according to the invention according to the invention with a carrier to a suitable recipient. A suitable recipient for example is a mammal, preferably human.

### Examples

### Example 1. Identification of a putative ligand for CD163, termed CD163L

The presence of the putative CD163 ligand (CD163L) on T cells was studied by labeling the soluble CD163 (or when available CD163L-specific antibodies) with FITC or biotin, incubating it with freshly isolated or cultured T cells or human umbillical vein endothelial cells (HUVEC), followed by fluorescence activated cell sorter (FACS) analysis. Using this technique, the regulation of the expression of the CD163L was studied. To this aim, freshly isolated or cultured T cells or HUVEC were stimulated with various cytokines and antibodies, followed by analysis of binding of the soluble CD163. When optimal conditions were characterized, T cells were lysed to isolate (membrane) proteins, and the CD163L protein was identified and (partly) sequenced by protein chemistry techniques (western blotting, 2D-SDS PAGE analysis, isoelectric focussing, immunoprecipitation, mass spectrometry, protein sequencing). In addition, absorption of sCD163 from a solution was studied by adding varying amounts of resting and activated T cells, and HUVEC. The soultion was subjected to SDS PAGE analysis, followed by western blotting using a CD163-specific antibody to show the absorption of sCD163 from the soultion. Reversely, the cells were lysed, and the proteins were subjected to SDS PAGE analysis, followed by western blotting using a CD163-specific antibody to that sCD163 was bound to the cells.

### Example 2. cDNA cloning, expression and purification of recombinant CD163L

The human CD163L was cloned using PCR primers based on (partial) amino acid sequences of CD163L as described in example 1. PCR reactions were performed on mRNA derived from T cells or HUVEC with CD163L expression, cloned into a plasmid vector, sequenced, amplified and expressed in several expression systems. Cloning and expression was carried out using standard techniques known to one of skill in the art. The CD163L was expressed and purified by affinity purification using sCD163 or anti-CD163L Mabs or by standard chromatography methods.

### Example 3. Generation of a soluble CD163L and a CD163L-Fc fusion protein.

CD163L and/or fractions thereof, and/or a sCD163L-Fc fusion protein can be generated to be used as therapeutic molecule. CD163L or truncated versions thereof comprising the CD163-binding site were generated and expressed. From these constructs, a highly soluble CD163L (sCD163) that could be expressed at high levels was isolated. Further, the sCD163L was expressed as fusion protein containing the extracellular part of the membrane molecules fused to the constant region of human IgG4. For the cloning of the human CH1/hinge-CH3 region PCR primers were designed based on the sequence by Ellison et al. (Ellison et al, 1982). To allow successful linkage of this fragment to the extracellular domains (ED) of CD163, a small linker of 6 amino acids was introduced. In this small linker the BamHI cloning site was incorporated to allow the in-frame fusion of the CD163 ED. The human IgG4 Fc-region was cloned from stimulated human B cells. Total RNA was isolated and by RT-PCR the Fc-region was cloned and sequence verified. For expression, the Fc region was subcloned in the baculovirus expression vector pVL1393. By DNA sequence analysis the construct was checked. Using suitable primers starting from activated human T cells the CD163L or a functional part thereof was amplified by RT-PCR. After sequence analysis the correct clones were cloned using BamHI in the already mentioned pVL1393 expression vector containing human Fc. About 3x10⁶ Sf9 insect cells were plated in a 25 cm² culture flask and co-transfected with 2 mg CD163L-Fc transfer vector together with 0.5 mg wild-type linearized baculovirus. After 3 days the virus was re-amplified on fresh Sf9 cells, followed by several new rounds to obtain recombinant baculovirus stocks for expression. With these virus stocks large scale infection were done on Sf9 cells. From these supernatant after 3 days both the CD163L-Fc protein was purified using a protein A column. Quality control was done by SDS-PAGE analysis.

To demonstrate the appropriate folding and activity of CD163L-Fc specific binding of CD163L-Fc to sCD163 was performed in an ELISA assay. Plates were coated with 50 ng/well of sCD163 or control, thereafter serial dilutions of CD163L-Fc and a control Fc-construct were applied to the plates. Detection was done by an anti-human kappa-light chain Mab labeled with alkaline phosphatase. Specific and dose-dependent binding was only observed in the titration curve in which sCD163 was combined with CD163L-Fc. All other incubation background absorbance was observed at all concentrations.

A sub-optimal concentration of the sCD163L was pre-incubated with a mixture of CD163L-Fc and a control Fc-construct, whereby one of the antigens was diluted from 1000 ng to 2 ng while the other antigen was maintained constant at 1000 ng. After macrophages were incubated with the pre-incubated material FACS analysis was performed. It was observed that when the control Fc-construct was kept at the same concentration and sCD163L-Fc was titrated binding of the CD163L occurred at high CD163L-Fc concentrations. Titration of the control Fc-construct did not decrease the MFI since high concentrations of CD163L remained available for binding to CD163. This also shows that the affinity of the CD163L-Fc for the CD163 was not different from the affinity for sCD163 present in human serum. Recombinant sCD163L-Fc fusion protein was purified from culture media by protein A or protein G.

### Example 4. In vitro immunomodulatory effect of sCD163L and sCD163L-Fc

The immunomodulatory effect of sCD163L and sCD163L-Fc was studied by performing a range of immunological assays. Mixed lymphocyte reactions (MLR), as well as antigen-specific T cell proliferation assays against antigens as tetanus toxoid, candida albicans, or house dust mite were performed in the absence or presence of sCD163L and/or sCD163L-Fc. These experiments showed that sCD163L and/or sCD163L-Fc enhanced the proliferation of allospecific (in the MLR) as well as antigen-specific T cell proliferation.

### Example 5. In vitro neutralization of immunosuppressive effect of CD163 by sCD163L and CD163L-Fc

The immunosuppressive effect of soluble was shown by MLR as well as by antigen-specific T cell proliferation assays (See example 21). Similar assays were performed in the absence or presence of sCD163L and CD163L-Fc. The results showed that the immunosuppressive effect of sCD163 was overcome by the addition of by sCD163L and CD163L-Fc to the cultures, most likely as a result of preventing the interaction of sCD163 with CD163L expressed on the T cells. This was proven to be correct in FACS experiment, in which labeled sCD163 was added to CD163L expressing T cells in the absence or presence of sCD163L and CD163L-Fc. Clearly, binding of sCD163 to CD163L on the T cells was inhibited by the addition of sCD163L and CD163L-Fc in a dose-dependent fashion.

### Example 6. In vivo neutralization of immunosuppressive effect of sCD163 by sCD163L and sCD163L-Fc

The neutralization of the immunosuppressive effects of sCD163 was tested in a mouse model of monocytic leukemia as described by Murosaki et al 2000. The murine monocyte/macrophage cell line P3881D1 was injected intraperitoneally into DBA/2 mice. In the control group this lead to a 100% mortality rate at day 25 after inoculation. However, survival was prolonged significantly by treatment with sCD163L and CD163L-Fc.
This experiment can be performed in a range of other relevant animal models that can be identified by those skilled in the art.

### Example 7. Treatment of human diseases in which serum levels of sCD163 are increased with sCD163L and sCD163L-Fc

A number of human diseases in which serum levels of sCD163 are increased are indications for treatment with sCD163L and/or sCD163L-Fc. These include but are not limited to lymphoma, myeloma, lymphotic leukemia, (myelo)monocytic leukemia, anemia, pneumonia and sepsis.

### Example 8. Stimulation of monocytes / macrophages via CD163 using sCD163L-Fc

Stimulation of CD163 expressing monocytes and/or macrophages using sCD163L-Fc was studied using human monocytes cultured in the presence of glucocorticoids to induce CD163 expression. The CD163-expressing monocytes were incubated in the presence of sCD163L-Fc. Supernatants of these cultures were collected, and the levels of cytokines produced (such as IL-6, IL-1b GMCSF and others) were determined, showing that the monocytes were activated by sCD163L-Fc. Further, cells were collected and the expression of a range of cell surface molecules (eg. CD14,CD40, MR, CD83 etc) was studied using FACS analysis. This analysis showed that the expression levels of a number of relevant cell surface markers was influenced as a result of sCD163L-Fc treatment.

### Example 9. Generation of antibodies to CD163L

Polyclonal antiserum against CD163L was raised by immunization of rabbits with recombinant human CD163L or fractions thereof (eg 4 injections/animal; 20-200 µg/injection). After the final booster the animals were bled to determine the titer of the polyclonal antiserum. To obtain monoclonal antibodies 6-8 wk old Balb/c mice were immunized with recombinant human CD163L or fractions thereof (for example 4 times with 2 wk intervals with 10-100 µg/injection dissolved in Freunds complete adjuvans for the first injection, and Freunds incomplete adjuvans for subsequent immunizations). Splenocytes were isolated and fused with a fusion cell line (Sp2/0 myeloma cells), followed by limiting dilution. Growing clones were screened using for example an enzyme-linked immunosorbant assay (ELISA). Therefore 96 wells plates were coated with CD163L or with a control protein. The culture supernatant was added, followed by washing and addition of a labeled anti-mouse antibody for detection. After limited dilution cloning of CD163L-specific antibody producing hybridomas stable hybridomas were obtained. From each clone cell supernatant was collected and by affinity chromatography using protein A sepharose columns (Pharmacia, Uppsala, Sweden) monoclonal antibodies were purified.

### Example 10. In vitro immunomodulatory effect of agonistic I and antagonistic I antibodies to CD163L

The immunomodulatory effect CD163L-specific agonistic I and antagonistic I antibodies was studied by performing a range of immunological assays. Mixed lymphocyte reactions (MLR), as well as antigen-specific T cell proliferation assays against antigens as tetanus toxoid, candida albicans, or house dust mite were performed in the absence or presence of CD163L-specific agonistic I and antagonistic I antibodies. These experiments showed that CD163L-specific agonistic antibodies inhibited the proliferation of allospecific (in the MLR) as well as antigen-specific T cell proliferation. In contrast, CD163L-specific antagonistic antibodies, an increased response was noted. Similar results were obtained when, the direct effect of agonistic I /antagonistic I antibodies to CD163L on T cell stimulation was studied by activating T cells with the cytokines as IL-2 and/or IL-15, by stimulation with mitogens as PMA, by stimulation with monoclonal antibodies (mAb) (for example with a combination of anti-CD28 and anti-CD3 mAb).

### Example 11. In vitro neutralization of immunosuppressive effect of CD163 by antagonistic I CD163L-specific antibodies

The immunosuppressive effect of soluble was shown by MLR as well as by antigen-specific T cell proliferation assays (See example 21). Similar assays were performed in the absence or presence of antagonistic I CD163L-specific antibodies. The results showed that the immunosuppressive effect of sCD163 was overcome by the addition of antagonistic I sCD163L-specific antibodies.

### Example 12. In vivo neutralization of immunosuppressive effect of sCD163 by antagonistic I CD163L-specific antibodies

The neutralization of the immunosuppressive effects of sCD163 by antagonistic I CD163L-specific antibodies was tested in a mouse model of monocytic leukemia as described by Murosaki et al. 2000. The murine monocyte/macrophage cell line P3881D1 was injected intraperitoneally into DBA/2 mice. In the control group this lead to a 100% mortality rate at day 25 after inoculation. However, survival was prolonged significantly by treatment with antagonistic I CD163L-specific antibodies. This experiment can be performed in a range of other relevant animal models that can be identified by those skilled in the art.

### Example 13. Treatment of human diseases in which serumlevels of sCD163 are increased with sCD163L and sCD163L-Fc

A number of human diseases in which serum levels of sCD163 are increased are indications for treatment with antagonistic I CD163L-specific antibodies. These include but are not limited to lymphoma, myeloma, lymphotic leukemia, (myelo)monocytic leukemia, anemia, pneumonia and sepsis.

### Example 14. In vivo immunosuppressive effect of agonistic CD163L-specific antibodies

The immunosuppressive activity of agonistic I CD163L-specific antibodies was tested in a mouse models for multiple sclerosis, namely acute Experimental Allergic Encephalomyelitis (EAE). EAE was induced with in SJL/J mice a synthetic peptide which is encephalitogenic in SJL/J. When mice were treated with agonistic I CD163L-specific antibody, efficacy was shown by decreased clinical symptoms of EAE as well as by histological analysis. Similar experiments can be performed by those skilled in the art in animal models for arthritis, asthma, transplantation, inflammatory bowel disease, and other inflammatory diseases.

### Example 15. Treatment of autoimmune and inflammatory diseases with agonistic I CD163L-specific antibodies

A variety and myriad number of autoimmune and inflammatory diseases are indications for treatment with agonistic I CD163L-specific antibodies of the invention. These include but are not limited to rheumatoid arthritis, diabetes, multiple sclerosis, systemic lupus erythematosous, psoriasis, autoimmune thyroiditis, asthma, inflammatory bowel disease, septic shock, transplant rejection, atherosclerosis, other cardiovascular diseases, and Alzheimer's disease .

### Example 16. Identification of CD163L expressing cells

Using the CD163L-specific antibodies described in example 9, the expression of CD163L on a wide variety of cell types was studies, by fluorescence activated cell sorter (FACS) analysis, and by immunohistochemistry. The expression of CD163L was shown on a number of cell types.

### Example 17. Assay to measure CD163L in biological fluids or culture supernatants

A method was developed in which an anti-CD163L monoclonal antibody or sCD163 is used to capture sCD163L. After this step, the sCD163L can be detected with another anti-CD163L mAb or sCD163 that are labeled to allow detection. This assay can be used as a diagnostic procedure, or can be used to screen compound for their effect on removal of CD163L from the cell membrane of CD163L-expressing cells.

### Example 18. Effect of agonistic I and antagonistic I CD163L-specific antibodies on degranulation of human basophils

As CD163L was shown to be present on basophils and mast cells, the effect of CD163L-specific agonistic I and antagonistic I antibodies on basophils that are triggered to degranulate either as a result of crosslinking of the high affinity receptor for IgE by anti IgE, or by stimulation with complement factor C5a was studied. It was demonstrated that the upregulation after activation as described above of two cell surface markers, namely CD203C (97A6) and CD63, was inhibited by agonistic I CD163L-specific antibodies. On the other hand, antagonistic I CD163L-specific antibodies did affect basophil degranulation.

### Example 19. Effect of antagonistic I and agonistic I CD163L-specific antibodies on adhesion of monocytes to endothelial cells

Glucocorticoid treated CD163 expressing monocytes have an increased adherance to human umbillical vein endothelial cells (HUVEC). The adherence of these monocytes to the HUVEC could be inhibited significantly by agonistic I as well as antagonistic I CD163L-specific antibodies.

### Example 20. Generation of a sCD163-Fc fusion protein

A cDNA encoding human CD163 was cloned from glucocorticoid stimulated monocytes using PCR primers based on the literature. From this full length clone, truncated variants were generated to generate a soluble CD163 (sCD163). The soluble CD163 was expressed in several expression systems, and purified. Recombinant sCD163-Fc fusion proteins were generated to be used as therapeutic molecule. CD163 was expressed as fusion protein containing the extracellular part of the membrane molecules fused to the constant region of human IgG4. For the cloning of the human CH1/hinge-CH3 region PCR primers were designed based on the published sequence. To allow successful linkage of this fragment to the extracellular domains (ED) of CD163, a small linker was introduced. In this small linker the BamHI cloning site was incorporated to allow the in-frame fusion of the CD163 ED. The human IgG4 Fc-region was cloned from stimulated human B cells. Total RNA was isolated and by RT-PCR the Fc-region was cloned and sequence verified. For expression, the Fc region was subcloned in the baculovirus expression vector pVL1393. Using suitable primers starting from human macrophages the extracellular domains of CD163 was amplified by RT-PCR and cloned into pUc18. A correct clone was sub cloned in the previously mentioned pVL1393 expression vector containing human Fc. About 3x10⁶ Sf9 insect cells were plated in a 25 cm² culture flask and co-transfected with 2 mg CD163EDFc transfer vector together with 0.5 mg wild-type linearized baculovirus. After 3 days the virus was re-amplified on fresh Sf9 cells, followed by several new rounds to obtain recombinant baculovirus stocks for expression. With these virus stocks large scale infection were done on Sf9 cells. From these supernatant after 3 days both the CD163EDFc protein was purified using a protein A column. Quality control was done by SDS-PAGE analysis.

To demonstrate the appropriate folding and activity of CD163-Fc specific binding of CD163-Fc to anti-CD163 and a control Mab was performed in an ELISA assay. Plates were coated with 50 ng/well of anti-CD163 or control Mab, thereafter serial dilutions of CD163-Fc and a control Fc-construct were applied to the plates. Detection was done by an anti-human kappa-light chain Mab labeled with alkaline phosphatase. Specific and dose-dependent binding was only observed in the titration curve in which anti-CD163 Mab was combined with CD163-Fc. All other incubation background absorbance was observed at all concentrations.

A sub-optimal concentration of the anti-CD163 Mab labeled with biotin was pre-incubated with a mixture of CD163-Fc and a control Fc-construct, whereby one of the antigens was diluted from 1000 ng to 2 ng while the other antigen was maintained constant at 1000 ng. After macrophages were incubated with the pre-incubated material FACS analysis was performed. It was observed that when the control Fc-construct was kept at the same concentration and CD163 was titrated binding of the anti-CD163-biotin occurred at low CD163-Fc concentrations. Titration of the control Fc-construct did not increase the MFI since all anti-CD163-biotin was complexed to the CD163-Fc. This also shows that the affinity of the anti-CD163 for the CD163-Fc was not different from the affinity for membrane-bound CD163 on human macrophages. Recombinant sCD163-Fc fusion protein was purified from culture media by protein A or protein G chromatography techniques.

### Example 21. Inhibition of antigen-specific T cell responses by recombinant soluble CD163 and sCD163-Fc fusion protein

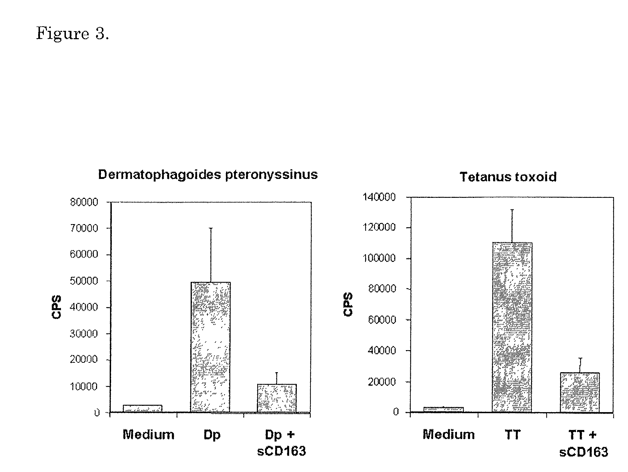

The immunosuppressive effect of sCD163 and sCD163-Fc were studied by performing a range of immunological assays. Mixed lymphocyte reactions were studied in the absence or presence of sCD163 and/or sCD163-Fc. Also, the influence of sCD163 and sCD163-Fc on antigen-specific T cell proliferation against antigens as tetanus toxoid, candida albicans, or house dust mite was studied. To this aim, peripheral blood mononuclear cells (PBMC) were isolated from a buffy coat by density centrifugation on lymphoprep (Nycomed), followed by three washes. The cells were suspended in IMDM, 5% human pool serum, and gentamycin as antibiotic, and 2x10⁵ PBMC were added per well to a 96 well round bottom tissue culture plate. To these wells was added tetantus toxoid (TT, final dilution 1:100, or house dust mite *Dermatophagoides pteronyssinus* (Dp, final dilution 1:50). To these cultures sCD163 and/or sCD163-Fc was added. Plates were cultured for 6 days, at the end of which the cultures were pulsed with BrDU labelling reagent (Cell proliferation ELISA BrdU chemoluminescence, Roche, Catalog No. 1 669 915). After overnight incubation, the incorporation of BrdU into proliferating cells was measured as indicated by the supplier. Surprisingly, whereas non-specific proliferation of T lymphocytes (cytokine-induced or PMA induced, see examples 32 and 33 was largely unaffected by the addition of sCD163, the TT- and Dp-induced T cell proliferation was inhibited by more than 75% by the addition of sCD163 (figure 3). More efficient immunosuppression was observed by the addition of sCD163-Fc.

### Example 22. Effect of sCD163 and sCD163-Fc on degranulation of human basophils

As CD163L was shown to be present on basophils and mast cells (see example 16), the effect of sCD163 on basophils that are triggered to degranulate either as a result of crosslinking of the high affinity receptor for IgE by anti IgE, or by stimulation with complement factor C5a was studied. It was demonstrated that the upregulation after activation as described above of two cell surface markers, namely CD203C (97A6) and CD63, was inhibited by the addition of sCD163.

### Example 23. Effect of sCD163 on adhesion of monocytes to endothelial cells

Glucocorticoid treated CD163 expressing monocytes have an increased adherance to human umbillical vein endothelial cells (HUVEC). The adherence of these monocytes to the HUVEC could be inhibited significantly by sCD163 and sCD163-Fc. Direct binding of sCD163 and sCD163-Fc to HUVEC was shown by FACS analysis, indicating that sCD163 and sCD163-Fc inhibit monocyte adherence to HUVEC by blocking the CD163L on HUVEC, making it unaccessible to the monocyte.

### Example 24. In vivo immunosuppressive effect of sCD163 and sCD163-Fc

The immunosuppressive activity of sCD163 and sCD163-Fc was tested in a mouse models for multiple sclerosis, namely acute Experimental Allergic Encephalomyelitis (EAE). EAE was induced with in SJL/J mice a synthetic peptide which is encephalitogenic in SJL/J. When mice were treated with sCD163 as well as with sCD163-Fc, efficacy was shown by decreased clinical symptoms of EAE as well as by histological analysis.
Similar experiments can be performed by those skilled in the art in animal models for arthritis, asthma, transplantation, inflammatory bowel disease, and other inflammatory diseases.

### Example 25. Treatment of autoimmune and inflammatory diseases with sCD163 and sCD163-Fc

A variety and myriad number of autoimmune and inflammatory diseases are indications for treatment with sCD163 and sCD163-Fc of the invention. These include but are not limited to rheumatoid arthritis, diabetes, multiple sclerosis, systemic lupus erythematosous, psoriasis, autoimmune thyroiditis, asthma, inflammatory bowel disease, septic shock, transplant rejection, atherosclerosis, other cardiovascular diseases, and Alzheimer's disease .

### Example 26. Screening assay to identify factors that induce or prevent CD163 shedding from cultured monocytes / macrophages

Human monocytes were cultured in the presence of glucocorticoids to induce CD163 expression. Upon induction, shedding of CD163 was induced by factors that one wishes to screen for an effect on CD163 shedding activity in the absence or presence of PMA. After incubation, supernatants were collected to measure sCD163 levels in western blotting analysis using commercially available anti-CD163 antibodies, and control cells and CD163-shedding cells were compared in FACS analysis using commercially available anti-CD163 antibodies. This method was used to identify factors that inhibited as well as factors that induced the shedding of sCD163.

### Example 27. Generation of antibodies to CD163

Polyclonal antiserum against sCD163 was raised by immunization of rabbits recombinant human sCD163 (eg 4 injections/animal; 20-200 µg/injection). After the final booster the animals are bled to determine the titer of the polyclonal antiserum. To obtain monoclonal antibodies 6-8 wk old Balb/c mice were immunized with human sCD163 (for example 4 times with 2 wk intervals with 10-100 µg/injection dissolved in Freunds complete adjuvans for the first injection, and Freunds incomplete adjuvans for subsequent immunizations). Splenocytes were isolated and fused with a fusion cell line such as Sp2/0 myeloma cells, followed by limiting dilution. Growing clones were screened using for example an enzyme-linked immunosorbant assay (ELISA). Therefore 96 wells plates were coated with soluble CD163 or with a control protein. The culture supernatant was added, followed by washing and addition of a labeled anti-mouse antibody for detection. After limited dilution cloning of sCD163-specific antibody producing hybridomas stable hybridomas were obtained. From each clone cell supernatant were collected and by affinity chromatography using protein A sepharose columns (Pharmacia, Uppsala, Sweden) monoclonal antibodies were purified. Monoclonal antibodies were further characterized by FACS analysis to determine binding of sCD163-specific mAbs to membrane bound CD163.

### Example 28. Stimulation of monocytes / macrophages using CD163-specific agonist II antibodies

Stimulation of CD163 expressing monocytes and/or macrophages using CD163-specific agonist II antibodies was studied using human monocytes cultured in the presence of glucocorticoids to induce CD163 expression. The CD163-expressing monocytes were incubated in the presence or absence of CD163-specific agonistic antibodies. Supernatants of these cultures were collected, and the levels of cytokines produced (such as IL-6, IL-1b, GMCSF and others) were determined, showing that the monocytes were activated by CD163-specific agonist II antibodies. Further, cells were collected and the expression of a range of cell surface molecules (eg. CD14,CD40, MR, CD83 etc) was studied using FACS analysis. This analysis showed that the expression levels of a number of relevant cell surface markers was influenced as a result of treatment with CD163-specific agonist II antibodies.

### Example 29. In vitro neutralization of immunosuppressive effect of CD163 by antagonist II or agonist II CD163-specific antibodies

The immunosuppressive effect of soluble CD163 was shown by MLR as well as by antigen-specific T cell proliferation assays (See example 21). Similar assays were performed in the absence or presence of agonistic II or antagonist II CD163-specific antibodies. The results showed that the immunosuppressive effect of sCD163 was overcome by the addition of antagonist II as well as agonist II sCD163-specific antibodies. Further, by staining CD163L-expressing cells with labeled sCD163 in the presence or absence of antagonist II or agonist II antibodies to CD163, it was demonstrated that the antibodies inhibited binding of sCD163 to CD163L.

### Example 30. In vivo neutralization of immunosuppressive effect of sCD163 by antagonist II or agonist II CD163-specific antibodies

The neutralization of the immunosuppressive effects of sCD163 by agonist II or antagonist II CD163-specific antibodies was tested in a mouse model of monocytic leukemia as described by Murosaki et al, 2000. The murine monocyte/macrophage cell line P3881D1 was injected intraperitoneally into DBA/2 mice. In the control group this lead to a 100% mortality rate at day 25 after inoculation. However, survival was prolonged significantly by treatment with antagonist II or agonist II CD163-specific antibodies. This experiment can be performed in a range of other relevant animal models that can be identified by those skilled in the art.

### Example 31. Treatment of human diseases in which serum levels of sCD163 are increased with agonist II or antagonist II antibodies to CD163

A number of human diseases in which serum levels of sCD163 are increased are indications for treatment with agonist II or antagonist II antibodies to CD163. These include but are not limited to lymphoma, myeloma, lymphotic leukemia, (myelo)monocytic leukemia, anemia, pneumonia and sepsis.

### Example 32. Inhibition of IL-2 induced T cell proliferation by recombinant soluble CD163 and sCD163-Fc fusion protein

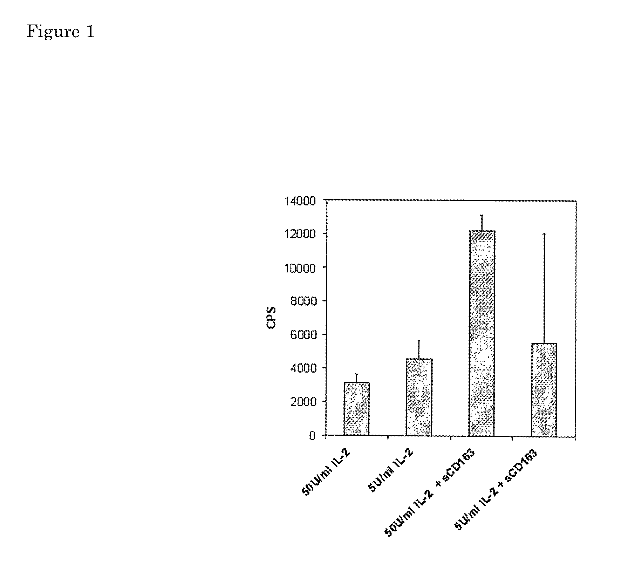

To examine the influence of sCD163 and sCD163-Fc on cytokine-induced T cell proliferation peripheral blood mononuclear cells (PBMC) were isolated from a buffy coat by density centrifugation on lymphoprep (Nycomed), followed by three washes. The cells were suspended in IMDM, 5% human pool serum, and gentamycin as antibiotic, and 2x10⁵ PBMC were added per well to a 96 well round bottom tissue culture plate. To these wells interleukin-2 (IL-2, 50 Units per ml or 5 units per ml as indicated). The cultures were performed in the absence or presence of sCD163. Plates were cultured for 6 days, at the end of which the cultures were pulsed with BrDU labelling reagent (Cell proliferation ELISA BrdU chemoluminescence, Roche, Catalog No. 1 669 915). After overnight incubation, the incorporation of BrdU into proliferating cells was measured as indicated by the supplier. As shown in figure 1 the addition of sCD163 enhanced, rather than decreased the IL-2 induced proliferation of T cells.

### Example 33. Inhibition of PMA-induced T cell proliferation by recombinant soluble CD163 and sCD163-Fc fusion protein

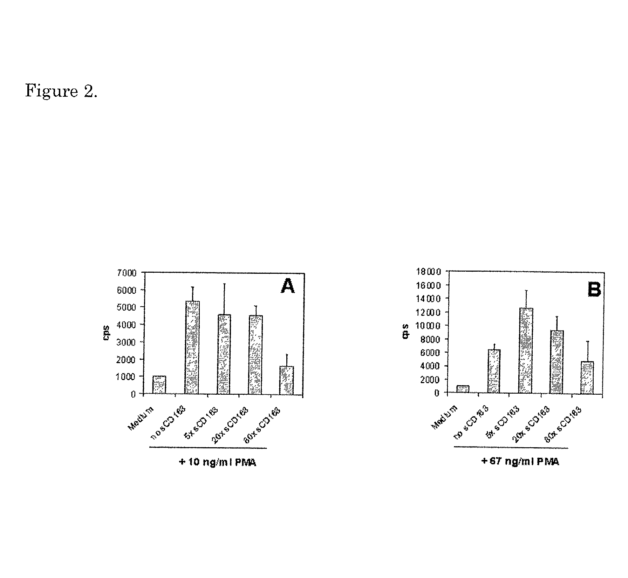

To examine the influence of sCD163 and sCD163-Fc on PMA-induced T cell proliferation peripheral blood mononuclear cells (PBMC) were isolated from a buffy coat by density centrifugation on lymphoprep (Nycomed), followed by treatment with lymfokwik-T to isolate T lymphocytes. The cells were suspended in IMDM, 5% human pool serum, and gentamycin as antibiotic, and 10⁵ T cells were added per well to a 96 well round bottom tissue culture plate. To these wells was added PMA at the concentration indicated in figure 2. The cultures were performed in the absence or presence of serial dilutions of sCD163. Plates were cultured for 24 hour, at the end of which the cultures were pulsed with BrDU labelling reagent (Cell proliferation ELISA BrdU chemoluminescence, Roche, Catalog No. 1 669 915). After overnight incubation, the incorporation of BrdU into proliferating T cells was measured as indicated by the supplier. As shown in figure 2, the addition of sCD163 at higher dilutions (e.g. 5x-20x sCD163) did not have a clear inhibitory effect on the PMA-induced proliferation of T lymphocytes. However, the addition of sCD163 at lower dilutions (e.g. 80x sCD163) did appear to have a slight inhibitory effect.

### Example 34. Determination of C- and N-terminus of sCD163.

To determine the exact C- and N-terminus of sCD163 shed from monocytes peripheral blood mononuclear cells (PBMC) were isolated from sixteen buffy coats by density centrifugation on lymphoprep, followed by percoll density centrifugation. The monocytes were collected from the interphase, washed and suspended in RPMI, 5% human pool serum, and gentamycin as antibiotic. The monocytes were cultured for 48h in the presence of 200mM dexamethasone.

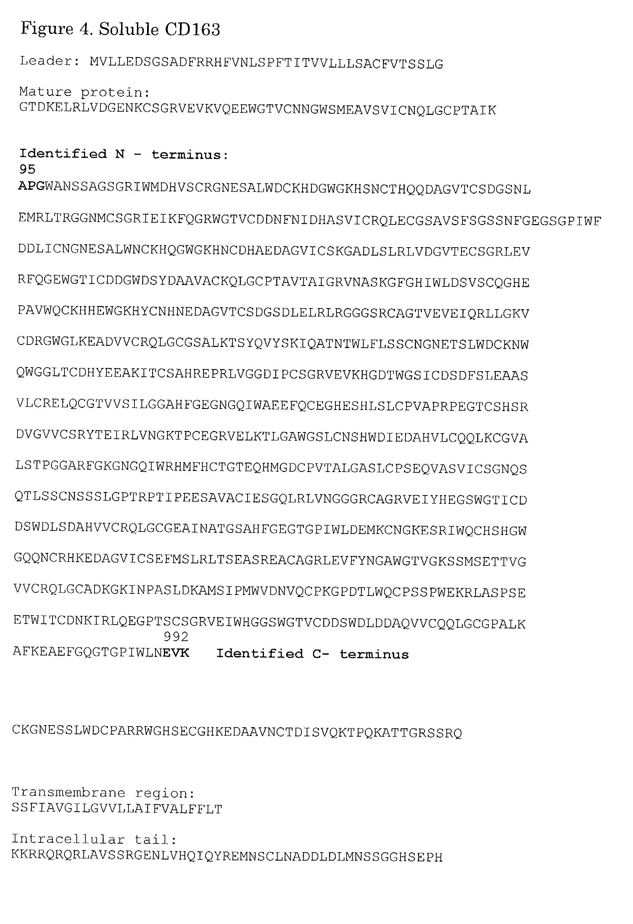

After 48 h, the cells were washed and resuspended in PBS (10⁷/ml). Shedding of membrane CD163 was induced by 50 nM PMA for 1,5 h at 37C. Cells were centrifuged and the supernatant containing sCD163 was removed and purified by ion exchange chromatography (IEX). For the determination of the C- and N-terminus, the sCD163 was subjected to electrophoresis on a denatured, reduced 7,5% SDS-PAGE gel, stained with coomassie blue, and the 130 kDa band (corresponding to the band of 110 kDa on the western blot made of a nonreduced SDS-PAGE) was excised for analysis of the N- and C-terminus by peptide mass fingerprinting. This analysis indicated that the 130 kDa (110 kDa under non-reducing circumstances) sCD163 was truncated at the C-terminus as well as at the N-terminus, resulting in a C-terminus of GPIWLNEVK (C terminal residue : lysine at 992) and a N-terminus of APGWANS (N terminal residue alanine 95)(figure 4). It should be noted however, that additional C- or N-terminal amino acids may be present but were not identified in the analysis.

### Example 35: Immunostimulatory effect of CD163-specific antibodies on antigen-specific T cell responses

The immunostimulatory effect of CD163-specific antibodies on antigen-specific T cell responses was studied against the antigens tetanus toxoid, and house dust mite. To this aim, peripheral blood mononuclear cells (PBMC) were isolated from a buffy coat by density centrifugation on lymphoprep (Nycomed), followed by three washes. The cells were suspended in IMDM, 5% human pool serum, and gentamycin as antibiotic, and 2x10⁵ PBMC were added per well to a 96 well round bottom tissue culture plate. To these wells was added tetantus toxoid (TT, final dilution 1:100), or house dust mite *Dermatophagoides pteronyssinus* (Dp, final dilution 1:50). To these cultures a mixture of three anti-CD163 mAbs was added at 5□/ml per antibody (GHI/61, RM3/1 and Ki-M8). In control wells, a mixture of three isotype-matched antibodies was added at 5 □g/ml per antibody. Plates were cultured for 6 days, at the end of which the cultures were pulsed with BrDU labelling reagent (Cell proliferation ELISA BrdU chemoluminescence, Roche, Catalog No. 1 669 915). After overnight incubation, the incorporation of BrdU into proliferating cells was measured as indicated by the supplier. Surprisingly, the TT- and Dp-induced T cell proliferation was strikingly enhanced by the addition of the CD163-specific antibodies, but not isotype control antibodies. This indicates that crosslinking of CD163 on the cell surface of leukocytes can result in enhanced immune activation. The antibodies did not induce T-cell proliferation in the absence of exogenous antigens.

### Figure legends

**Figure 1.** To examine the influence of sCD163 and sCD163-Fc on cytokine-induced T cell proliferation peripheral blood mononuclear cells (PBMC) were isolated from a buffy coat by density centrifugation on lymphoprep (Nycomed), followed by three washes. The cells were suspended in IMDM, 5% human pool serum, and gentamycin as antibiotic, and 2x10⁵ PBMC were added per well to a 96 well round bottom tissue culture plate. To these wells interleukin-2 (IL-2 , 50 Units per ml or 5 units per ml as indicated). The cultures were performed in the absence or presence of sCD163. Plates were cultured for 6 days, at the end of which the cultures were pulsed with BrDU labelling reagent (Cell proliferation ELISA BrdU chemoluminescence, Roche, Catalog No. 1 669 915). After overnight incubation, the incorporation of BrdU into proliferating cells was measured as indicated by the supplier. As shown in figure 1 the addition of sCD163 enhanced, rather than decreased the IL-2 induced proliferation of T cells.

**Figure 2.** To examine the influence of sCD163 and sCD163-Fc on PMA-induced T cell proliferation peripheral blood mononuclear cells (PBMC) were isolated from a buffy coat by density centrifugation on lymphoprep (Nycomed), followed by treatment with lymfokwik-T to isolate T lymphocytes. The cells were suspended in IMDM, 5% human pool serum, and gentamycin as antibiotic, and 10⁵ T cells were added per well to a 96 well round bottom tissue culture plate. To these wells was added PMA at the concentration indicated in figure 2. The cultures were performed in the absence or presence of serial dilutions of sCD163. Plates were cultured for 24 hour, at the end of which the cultures were pulsed with BrDU labelling reagent (Cell proliferation ELISA BrdU chemoluminescence, Roche, Catalog No. 1 669 915). After overnight incubation, the incorporation of BrdU into proliferating T cells was measured as indicated by the supplier. As shown in figure 2, the addition of sCD163 did not have a clear inhibitory effect on the PMA-induced proliferation of T lymphocytes.

**Figure 3.** Inhibition of antigen-specific T-cell proliferation by sCD163.

Peripheral blood mononuclear cells (PBMC) were isolated from a buffy coat by density centrifugation on lymphoprep (Nycomed), followed by three washes. The cells were suspended in IMDM, 5% human pool serum, and gentamycin as antibiotic, and 2x10⁵ PBMC were added per well to a 96 well round bottom tissue culture plate. To these wells was added tetantus toxoid (TT, final dilution 1:100, or house dust mite *Dermatophagoides pteronyssinus* (Dp, final dilution 1:50). To these cultures a preparation of sCD163, shed from dexamethasone treated monocytes and purified by ion exchange chromatography was added (5 times diluted). Plates were cultured for 6 days, at the end of which the cultures were pulsed with BrDU labelling reagent (Cell proliferation ELISA BrdU chemoluminescence, Roche, Catalog No. 1 669 915). After overnight incubation, the incorporation of BrdU into proliferating cells was measured as indicated by the supplier. Results are shown as counts per second. Surprisingly, whereas non-specific proliferation of T lymphocytes (cytokine-induced or PMA induced, see examples 32 and 33) was largely unaffected by the addition of sCD163, the TT- and Dp-induced T cell proliferation was inhibited by more than 75% by the addition of sCD163. Similar results were obtained by the addition of sCD163-Fc.

**Figure 4.** The amino acid sequence of soluble CD163 as deduced by peptide mass fingerprinting.

### REFERENCES

Murosaki S, Muroyama K, Yamamoto Y, Yoshikai Y. Antitumor effect of heat-killed Lactobacillus plantarum L-137 through restoration of impaired interleukin-12 production in tumor-bearing mice. Cancer Immunol Immunother 2000 49:157-64.
Guyre PM, Morganelli PM, Goulding NJ. Methods for detecting inflammation and inflammatory conditions. WO 01/73435.
Guyre PM, Morganelli PM, Goulding NJ. Methods for detecting inflammation and inflammatory conditions. US 20010041177.
Morganelli PM, Guyre PM. Monoclonal antibodies specific for a human mononuclear phagocyte-specific antigen US 5077216.
Baeten D et al. 2002. J Pathology. 196: 343-350.
Buechler C, Ritter M, Orso E, Langmann T, Klucken J, Schmitz G. Regulation of scavenger receptor CD163 expression in human monocytes and macrophages by pro- and antiinflammatory stimuli. J Leukoc Biol. 2000;67:97-103.
Droste A, Sorg C, Hogger P. Shedding of CD163, a novel regulatory mechanism for a member of the scavenger receptor cysteine-rich family. Biochem Biophys Res Commun. 1999;256:110-3.
Ellison JW, Berson BJ, Hood LE. The nucleotide sequence of a human immunoglobulin C gammal gene. Nucleic Acids Res. 1982 Jul 10;10(13):4071-9. van den Heuvel MM, Tensen CP, van As JH, Van den Berg TK, Fluitsma DM, Dijkstra CD, Dopp EA, Droste A, Van Gaalen FA, Sorg C, Hogger P, Beelen RH. Regulation of CD 163 on human macrophages: cross-linking of CD163 induces signaling and activation. J Leukoc Biol. 1999;66:858-66.
Hogger P, Dreier J, Droste A, Buck F, Sorg C. Identification of the integral membrane protein RM3/1 on human monocytes as a glucocorticoid-inducible member of the scavenger receptor cysteine-rich family (CD163).J Immunol. 1998 ;161:1883-90.
Hogger P, Sorg C. Soluble CD163 inhibits phorbol ester-induced lymphocyte proliferation.
   Biochem Biophys Res Commun. 2001;288:841-3.
Imler JL, Hoffmann JA. Toll receptors in innate immunity. Trends Cell Biol. 2001;11:304-11.
Kopp EB, Medzhitov R. The Toll-receptor family and control of innate immunity.
   Curr Opin Immunol. 1999;11:13-8
Kristiansen M, Graversen JH, Jacobsen C, Sonne O, Hoffman HJ, Law SK, Moestrup SK. Identification of the haemoglobin scavenger receptor. Nature. 2001;409:198-201.
Law SK, Micklem KJ, Shaw JM, Zhang XP, Dong Y, Willis AC, Mason DY. A new macrophage differentiation antigen which is a member of the scavenger receptor superfamily. Eur J Immunol. 1993;23:2320-5.
Linehan SA, Martinez-Pomares L, Gordon S. Mannose receptor and scavenger receptor: two macrophage pattern recognition receptors with diverse functions in tissue homeostasis and host defense. Adv Exp Med Biol. 2000;479:1-14.
Möller HJ, Peterslund NA, Graversen JH, Moestrup SK. Identification of the hemoglobin scavenger receptor/CD163 as a natural soluble protein in plasma. Blood. 2002 ;99:378-80.
Morganelli PM, Guyre PM. IFN-gamma plus glucocorticoids stimulate the expression of a newly identified human mononuclear phagocyte-specific antigen. J Immunol. 1988;140:2296-304.
Polfliet MM, van de Veerdonk F, Dopp EA, van Kesteren-Hendrikx EM, van Rooijen N, Dijkstra CD, van den Berg TK. The role of perivascular and meningeal macrophages in experimental allergic encephalomyelitis. J Neuroimmunol. 2002 Jan;122(1-2):1-8.
Pulford K, Micklem K, McCarthy S, Cordell J, Jones M, Mason DY. A monocyte/macrophage antigen recognized by the four antibodies GHI/61, Ber-MAC3, Ki-M8 and SM4. Immunology. 1992;75:588-95
Re F, Strominger JL. Toll-like receptor 2 (TLR2) and TLR4 differentially activate human dendritic cells. J Biol Chem. 2001;276:37692-9. Ritter M, Buechler C, Langmann T, Schmitz G. Genomic organization and chromosomal localization of the human CD163 (M130) gene: a member of the scavenger receptor cysteine-rich superfamily. Biochem Biophys Res Commun. 1999;260:466-74.
Ritter M, Buechler C, Langmann T and Schmitz G. Genomic organization and chromosomal localization of the human CD163 (M130) gene: a member of the scavenger receptor cysteine-rich Superfamily Biochem. Biophys. Res. Commun. 260, 466-474 (1999)
Ritter M, Buechler C, Kapinsky M, Schmitz G. Interaction of CD163 with the regulatory subunit of casein kinase II (CKII) and dependence of CD163 signaling on CKII and protein kinase C. Eur J Immunol. 2001;31:999-1009.
Schaer DJ, Boretti FS, Hongegger A, Poehler D, Linnscheid P, Staege H, Muller C, Schoedon G, Schaffner A. Molecular cloning and characterization of the mouse CD163 homologue, a highly glucocorticoid-inducible member of the scavenger receptor cysteine-rich family. Immunogenetics. 2001 53:170-7.
Sulahian TH, Hogger P, Wahner AE, Wardwell K, Goulding NJ, Sorg C, Droste A, Stehling M, Wallace PK, Morganelli PM, Guyre PM. Human monocytes express CD163, which is upregulated by IL-10 and identical to p155. Cytokine. 2000;12:1312-21.
Sulahian TH, Hintz KA, Wardwell K, Guyre PM. Development of an ELISA to measure soluble CD163 in biological fluids.J Immunol Methods. 2001 Jun 1;252(1-2):25-31.
Zwadlo G, Voegeli R, Osthoff KS, Sorg C. A monoclonal antibody to a novel differentiation antigen on human macrophages associated with the down-regulatory phase of the inflammatory process. Adv. Exp Cell Biol. 1987;55:295-304
Zwadlo-Klarwasser G, Bent S, Haubeck HD, Sorg C, Schmutzler W. Glucocorticoid-induced appearance of the macrophage subtype RM 3/1 in peripheral blood of man.
   Int Arch Allergy Appl Immunol. 1990;91(2):175-80.

## Claims

1. A method for identifying a molecule with immune modulatory activity capable of interacting with CD163 comprising providing CD163 or a functional part, derivative and/or analogue thereof and under suitable conditions detecting a molecule capable of interacting with said CD163 and determining whether said molecule is capable of modulating an immune response.

2. A method according to claim 1 wherein said CD163 comprises a soluble CD163.

3. A method according to claim 1 or claim 2 wherein said molecule with immune modulatory activity is cell bound and/or soluble.

4. A method according to anyone of claims 1-3, wherein said molecule comprises a proteinaceous molecule, functional derivative, functional fragment and/or analogue thereof.

5. An isolated and/or recombinant and/or synthetic molecule obtainable by a method according to anyone of claims 1-4.

6. A molecule according to claim 5 which comprises a receptor or functional fragment thereof.

7. A molecule according to claim 5 or claim 6 coupled to a moiety.

8. Use of a molecule according to anyone of claims 5-7 to modulate an immune response.

9. Use according to claim 8 wherein said modulation comprises augmentation of an immune response.

10. Use according to claim 8 wherein said modulation comprises suppression of an immune response.

11. An antagonist I of a molecule according to anyone of claims 5-7.

12. An antagonist I according to claim 11 which is an antibody or functional part, derivative and/or analogue thereof.

13. An antagonist I according to claim 11 or claim 12 coupled to a moiety.

14. Use of an antagonist I according to anyone of claims 11-13 to modulate an immune response.

15. Use according to claim 14 wherein said modulation comprises augmentation of an immune response.

16. An agonist I of a molecule according to anyone of claims 5-7.

17. An agonist I according to claim 16 which is an antibody or functional part, derivative and/or analogue thereof.

18. An agonist I according to claim 16 or claim 17 coupled to a moiety.

19. Use of an agonist I according to anyone of claim 16-18 to modulate an immune response.

20. Use according to claim 19 wherein said modulation comprises suppression of an immune response.

21. An isolated and/or recombinant and/or synthetic CD163, a functional part, derivative and/or analogue thereof.

22. An isolated CD163 according to claim 21, wherein said CD163 comprises a soluble CD163.

23. An isolated CD163 according to claim 21 or claim 22 coupled to a moiety.

24. An isolated CD163 according to claim 23 wherein said moiety comprises a constant region of an immunoglobulin.

25. An antagonist II of CD163.

26. An antagonist II according to claim 25 which is an antibody or functional part, derivative and/or analogue thereof.

27. An antagonist II according to claim 25 or claim 26 coupled to a moiety.

28. An agonist II of CD163.

29. An agonist II according to claim 28 which is an antibody or functional part, derivative and/or analogue thereof.

30. An agonist II according to claim 28 or claim 29 coupled to a moiety.

31. A method to detect the presence of a molecule according to anyone of claims 5-7 in a sample comprising; contacting the sample with a binding molecule for said molecule to form a complex; further comprising detecting said complex in the sample.

32. A method according to claim 31, wherein said binding molecule comprises an antagonist I according to anyone of claims 11-13 or an agonist I according to anyone of claim 16-18.

33. A method according to claim 31, wherein said binding molecule comprises membrane bound CD163 and/or a soluble CD163.

34. A method to determine the binding activity of a molecule according to anyone of claims 5-7 in a sample comprising detecting the presence of a molecule using a method according to anyone of claims 31-33, further determining the levels of binding molecule-molecule in said sample.

35. A nucleic acid, functional part, functional derivative and/or analogue thereof encoding a molecule according to anyone of claims 5-7.

36. A nucleic acid, functional part, functional derivative and/or analogue thereof encoding an antagonist I of a molecule according to claim 11-13.

37. A nucleic acid, functional part, functional derivative and/or analogue thereof encoding an agonist I of a molecule according to anyone of claim 16-18.

38. A nucleic acid functional part, functional derivative and/or analogue thereof encoding an isolated CD163 according to anyone of claims 21-24.

39. A nucleic acid functional part, functional derivative and/or analogue thereof encoding an antagonist II of CD 163 according to anyone of claims 25-27.

40. A nucleic acid functional part, functional derivative and/or analogue thereof encoding an agonist II of CD163 according anyone of claims 28-30.

41. A vector comprising a nucleic acid according to anyone of claims 35-40.

42. A cell comprising a vector according to claim 41.

43. A gene delivery vehicle comprising a vector according to claim 41.

44. A method for the production of a molecule according to anyone of claims 5-7, or an antagonist I of said molecule according to anyone of claims 11-13 or an agonist I of said molecule according to anyone of claims 16-18, or an isolated CD163 according to anyone of claims 21-24, or an antagonist II of CD163 according to anyone of claims 25-27 or an agonist II of CD163 according to anyone of claims 28-30 in an organism comprising inserting into the genome of said organism one or more copies of a nucleic acid according to anyone of claims 35-40.

45. Use of an isolated CD163 according to anyone of claims 21-24 and/or an antagonist II of CD163 according to anyone of claims 25-27 and/or an agonist II of CD163 according to anyone of claims 28-30 to modulate an immune response.

46. Use according to claim 45, wherein said immune response comprises an antigen specific immune response.

47. A pharmaceutical composition comprising a molecule according to anyone of claims 5-7, and/or an antagonist I of said molecule according to anyone of claims 11-13 and/or an agonist I of said molecule according to anyone of claims 16-18, and/or an isolated CD163 according to anyone of claims 21-24, and/or an antagonist II of CD163 according to anyone of claims 25-27, and/or an agonist II of CD163 according to anyone of claims 28-30 and/or a cell according to claim 42 and/or a gene delivery vehicle according to claim 43.

48. A pharmaceutical composition according to claim 47 for augmenting or suppressing an immune response.

49. Use of a molecule according to anyone of claims 5-7, and/or an antagonist I of said molecule according to anyone of claims 11-13 and/or an agonist I of said molecule according to anyone of claims 16-18, and/or an isolated CD163 according to anyone of claims 21-24, and/or an antagonist II of CD163 according to anyone of claims 25-27, and/or an agonist II of CD163 according to anyone of claims 28-30 and/or a cell according to claim 42 and/or a gene delivery vehicle according to claim 43 in the preparation of a pharmaceutical composition for the treatment and/or prophylactic treatment of a disease, treatable by modulating an immune response.

50. Use according to claim 49, wherein said disease comprises an autoimmune disease, Hepatitis, allergy, asthma, inflammatory disease, cancer, infectious diseases, host versus graft related diseases, cardiovascular disease and/or neurological diseases and/or a disease associated with elevated serum sCD163 levels.

51. A method of treatment of a immune related disease comprising administrating a molecule according to anyone of claims 5-7, and/or an antagonist I of said molecule according to anyone of claims 11-13 and/or an agonist I of said molecule according to anyone of claims 16-18, and/or an isolated CD163 according to anyone of claims 21-24, and/or an antagonist II of CD163 according to anyone of claims 25-27, and/or an agonist II of CD163 according to anyone of claims 28-30 and/or a cell according to claim 42 and/or a gene delivery vehicle according to claim 43 with a carrier to a suitable recipient.
